# EUROPEAN PATENT SPECIFICATION

(11) **EP 3 989 301 B1**
(45) Date of publication and mention of the grant of the patent: **28.10.2026**
(21) Application number: 20203460.9
(22) Date of filing: 22.10.2020
(51) Int. Cl.: C07C 255/51, C07D 213/61, H10K 85/60, H10K 50/17, H10K 50/81

(54) **ORGANIC ELECTRONIC DEVICE COMPRISING A COMPOUND OF FORMULA (1), DISPLAY DEVICE COMPRISING THE ORGANIC ELECTRONIC DEVICE**
ORGANISCHE ELEKTRONISCHE VORRICHTUNG MIT EINER VERBINDUNG DER FORMEL (1), ANZEIGEVORRICHTUNG MIT DER ORGANISCHEN ELEKTRONISCHEN VORRICHTUNG
DISPOSITIF ÉLECTRONIQUE ORGANIQUE COMPRENANT UN COMPOSÉ DE FORMULE (1), DISPOSITIF D'AFFICHAGE COMPRENANT LE DISPOSITIF ÉLECTRONIQUE ORGANIQUE

(43) Date of publication of application: 27.04.2022
(73) Proprietor: Novaled GmbH, 01099 Dresden (DE)
(72) Inventor: NÜLLEN, Max Peter, 01099 Dresden (DE); SCHULZE, Benjamin, 01099 Dresden (DE); WUDARCZYK, Jakob Jacek, 01099 Dresden (DE); LUSCHTINETZ, Regina, 01099 Dresden (DE); LANGGUTH, Oliver, 01099 Dresden (DE)
(74) Representative: Michalski Hüttermann & Partner mbB

(56) References cited:
- EP-A1- 1 988 587
- EP-A1- 3 416 207
- WO-A1-2019/168368
- US-A1- 2011 127 500

## Description

### Technical Field

The present invention relates to an organic electronic device comprising a compound of formula (1) and a display device comprising the organic electronic device.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of metal complexes which are also contained in the semiconductor layer.

There remains a need to improve performance of organic semiconductor materials, semiconductor layers, as well as organic electronic devices thereof, in particular to achieve improved operating voltage stability over time through improving the characteristics of the compounds comprised therein. WO2019/168368 (A1) discloses OLED devices comprising a radialene compouns in the hole injection layer. US2011/127500 (A1) discloses OLED devices with composite ITO/Ag/ITO anode electrodes.

### DISCLOSURE

An aspect of the present invention provides an organic electronic device according to the claims comprising a substrate, an anode layer, a cathode layer, at least one first emission layer, and a hole injection layer, wherein the hole injection layer is arranged between the first emission layer and the anode layer,
and whereby the hole injection layer comprises a compound of formula (I) 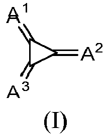
whereby A¹ is selected from formula (II) 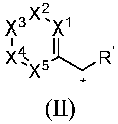
X¹ is selected from CR¹ or N;
X² is selected from CR² or N;
X³ is selected from CR³ or N;
X⁴ is selected from CR⁴ or N;
X⁵ is selected from CR⁵ or N;
R¹, R², R³, R⁴ and R⁵ (if present) are independently selected from CN, partially fluorinated or perfluorinated C₁ to C₈ alkyl, halogen, Cl, F, D or H, whereby when any of R¹, R², R³, R⁴ and R⁵ is present, then the corresponding X¹, X², X³, X⁴ and X⁵ is not N;
with the proviso that at least two of the following requirements a) to e) are fulfilled:
   a) At least one R¹, R², R³, R⁴ and R⁵ is independently selected from CN, partially flurorinated or perfluorinated C₁ to C₈ alkyl, halogen, Cl, F, and at least one remaining R¹, R², R³, R⁴ and R⁵ is selected D or H;
   b) R¹ or R² are selected from CN or partially flurorinated or perfluorinated C₁ to C₈ alkyl and at least one remaining R¹ to R⁵ is independently selected from CN, partially flurorinated or perfluorinated C₁ to C₈ alkyl, halogen, Cl or F;
   c) R3 is selected from partially flurorinated or perfluorinated C₁ to C₈ alkyl and at least one of R¹, R², R⁴ and R⁵ is independently selected from CN, partially flurorinated or perfluorinated C₁ to C₈ alkyl, halogen, Cl or F;
   d) at least two R¹ to R⁵ are independently selected from CN or partially flurorinated or perfluorinated C₁ to C₈ alkyl; or
   e) at least one X¹ to X⁵ is N and at least two X¹ to X⁵ are selected from CR¹ to CR⁵;
A² and A³ are independently selected from formula (III) 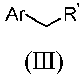
wherein Ar is independently selected from substituted or unsubstituted C₆ to C₁₈ aryl and substituted or unsubstituted C₂ to C₁₈ heteroaryl, wherein the substituents on Ar are independently selected from CN, partially or perfluorinated C₁ to C₆ alkyl, halogen, Cl, F, D; and
R' is selected from Ar, substituted or unsubstituted C₆ to C₁₈ aryl or C₃ to C₁₈ heteroaryl, partially flurorinated or perfluorinated C₁ to C₈ alkyl, halogen, F or CN;
and whereby the anode layer comprises a first anode sub-layer and a second anode sub-layer, wherein
   - the first anode sub-layer comprises a first metal having a work function in the range of ≥ 4 and ≤ 6 eV, wherein the first metal of the first anode sub-layer is selected from the group comprising Ag, and
   - the second anode sub-layer comprises a transparent conductive oxide; and
   - the second anode sub-layer is arranged closer to the hole injection layer;
   - wherein the anode layer of the organic electronic device comprises in addition a third anode sub-layer comprising a transparent conductive oxide, wherein the third anode sub-layer is arranged between the substrate and the first anode sub-layer;
   - whereby the transparent conductive oxide is selected from the group comprising indium tin oxide.

It should be noted that throughout the application and the claims any Aⁿ, Bⁿ, Rⁿ etc. always refer to the same moieties, unless otherwise noted.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

### Advantageous Effects

Surprisingly, it was found that the organic electronic device according to the invention solves the problem underlying the present invention by enabling devices in various aspects superior over the organic electroluminescent devices known in the art, in particular with respect to operating voltage over lifetime.

According to one embodiment of the present invention, the first metal of the first anode sub-layer is Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide

According to one embodiment of the present invention, the second anode sub-layer may has a thickness in the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to the present invention, anode layer of the organic electronic device comprises in addition a third anode sub-layer comprising a transparent conductive oxide, wherein the third anode sub-layer is arranged between the substrate and the first anode sub-layer.

According to the present invention, the third anode sub-layer comprises indium tin oxide.

According to one embodiment of the present invention, the third anode sub-layer may has a thickness in the range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the hole injection layer is in direct contact with the anode layer.

According to one embodiment of the present invention, the hole injection layer comprises a compound of formula (IV) 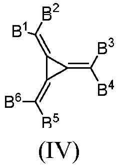
whereby B¹ is selected from formula (V) 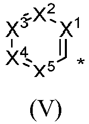
B³ and B⁵ are Ar and B², B⁴ and B⁶ are R'.

According to the present invention, at least two of the requirements a) to e) are fulfilled.

According to one embodiment of the present invention, A² and A³ are identical

According to one embodiment of the present invention, A¹ differs from A² and A³.

According to one embodiment of the present invention, at least one from A² and A³ is identical to A¹.

According to one embodiment, the hole injection layer comprises a composition comprising a compound of formula (IV) and at least one compound of formula (IVa) to (IVd)

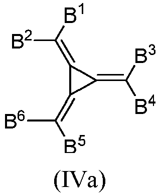

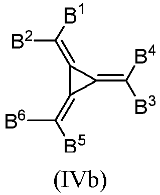

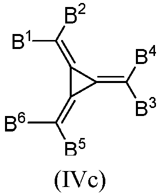

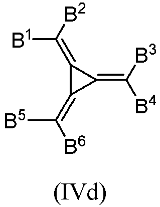

In case the hole injection layer comprises such a composition, throughout this application text the term "compound of formula (I)" shall also intend to include the composition as described above.

According to one embodiment of the present invention, in formula (II) at least one of X¹, X² and X³ is selected from CH.

According to one alternative embodiment of the present invention, in formula (II) two of X¹, X² and X³ are selected from CH.

According to one embodiment of the present invention, X¹ and X² are independently selected from CH or N and X³ is selected from CH.

According to one embodiment of the present invention, R¹ is preferably selected from perfluorinated C₁ to C₆ alkyl or CN, more preferably perfluorinated C₁ to C₄ alkyl or CN, even more preferred CF₃ or CN, further preferred CF₃.

According to one embodiment of the present invention, R² is preferably selected from perfluorinated C₁ to C₆ alkyl, more preferably perfluorinated C₁ to C₄ alkyl, even more preferred CF₃.

According to one embodiment of the present invention, R³ is selected from CN, partially or fully fluorinated C₁ to C₄ alkyl, partially or fully fluorinated C₁ to C₄ alkoxy, substituted or unsubstituted C₆ to C₁₂ aryl or C₃ to C₁₂ heteroaryl, wherein the substituents are selected from halogen, F, Cl, CN, partially or fully fluorinated C₁ to C₄ alkyl, partially or fully fluorinated C₁ to C₄ alkoxy; more preferred R³ is selected from CN, CF₃, OCF₃ or F, most preferred CN.

According to one embodiment of the present invention, R³ is selected from CN or partially flurorinated or perfluorinated C₁ to C₈ alkyl and one R¹, R², R⁴, R⁵ is selected from H or D; preferably R³ is selected from CN or partially flurorinated or perfluorinated C₁ to C₄ alkyl and one R¹, R², R⁴, R⁵ is selected from H or D; alternatively R³ is selected from CN or partially CF₃ and one R¹, R², R⁴, R⁵ is selected from H or D.

According to one embodiment of the present invention, R³ is selected from CN or partially flurorinated or perfluorinated C₁ to C₈ alkyl and at least one R¹, R², R⁴, R⁵ is selected from H or D; preferably R³ is selected from CN or partially flurorinated or perfluorinated C₁ to C₄ alkyl and at least one R¹, R², R⁴, R⁵ is selected from H or D; alternatively R³ is selected from CN or partially CF₃ and at least one R¹, R², R⁴, R⁵ is selected from H or D.

According to one embodiment of the present invention, R³ is selected from CN or partially flurorinated or perfluorinated C₁ to C₈ alkyl and two or three R¹, R² , R⁴, R⁵ is selected from H or D; preferably R³ is selected from CN or partially flurorinated or perfluorinated C₁ to C₄ alkyl and two or three R¹, R², R⁴, R⁵ is selected from H or D; alternatively R³ is selected from CN or partially CF₃ and two or three R¹, R², R⁴, R⁵ is selected from H or D.

According to one embodiment of the present invention, at least one X¹ to X⁵ is N and at least one R¹ to R⁵ is selected from CN, partially flurorinated or perfluorinated C₁ to C₈ alkyl, halogen, Cl, F.

According to one embodiment of the present invention, Ar is selected from substituted or unsubstituted C₆ to C₁₂ aryl and substituted or unsubstituted C₃ to C₁₂ heteroaryl, wherein the substituents on Ar are independently selected from CN, partially or perfluorinated C₁ to C₄ alkyl, halogen, F; preferably Ar is selected from substituted phenyl, pyridyl, pyrimidyl or triazinyl, wherein the substituents on Ar are independently selected from CN, CF₃ or F.

According to one embodiment of the present invention, A² is selected from formula (IIIa) 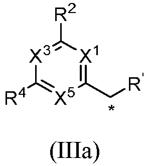 and A³ is selected from formula (III).

According to one alternative embodiment of the present invention, A² and A³ are independently selected from formula (IIIa).

According to one embodiment of the present invention, A¹, A² and A³ are selected the same.

According to one embodiment of the present invention, A² and A³ are selected the same and A¹ is selected differently from A² and A³.

According to one embodiment of the present invention, A¹ and A² are selected the same and A³ is selected differently from A¹ and A².

According to one embodiment of the present invention, R' is CN.

According to one embodiment of the present invention, formula (II) is selected from the group comprising the following moieties:

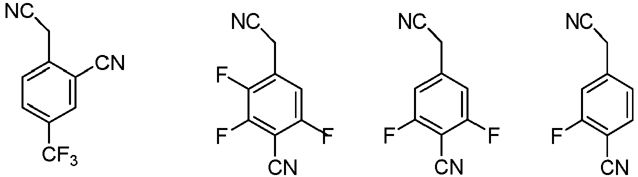

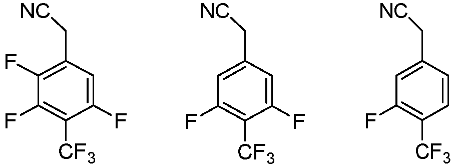

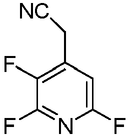

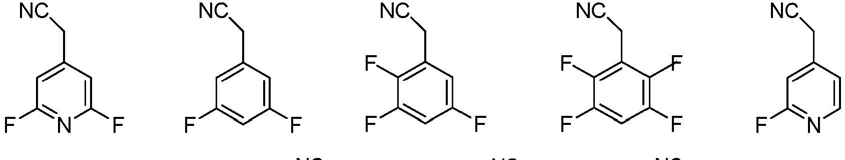

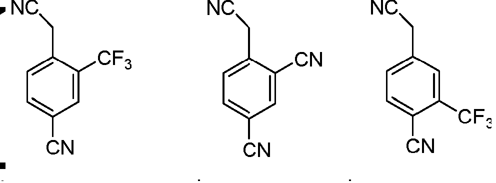

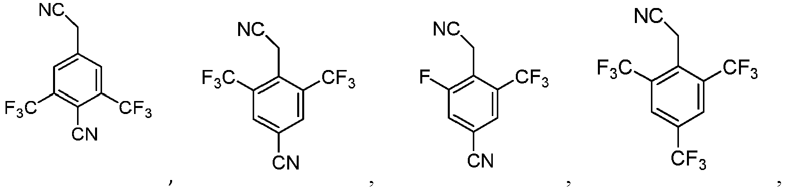

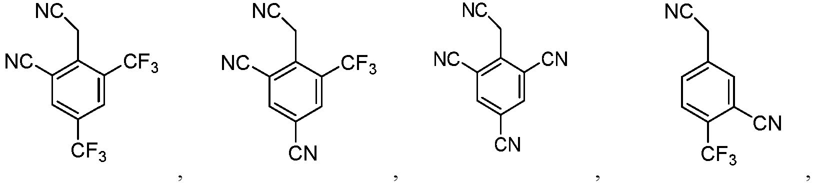

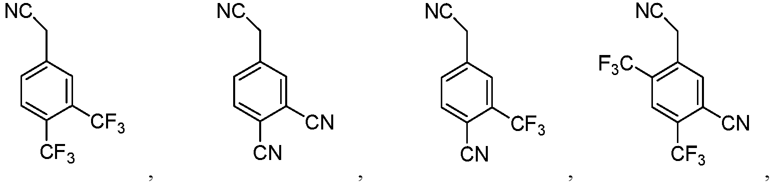

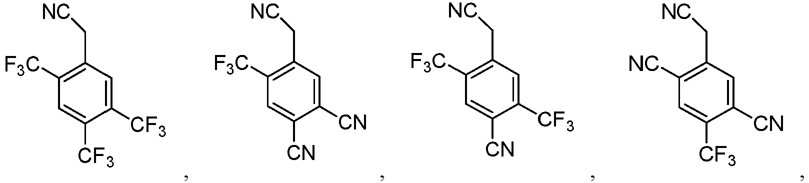

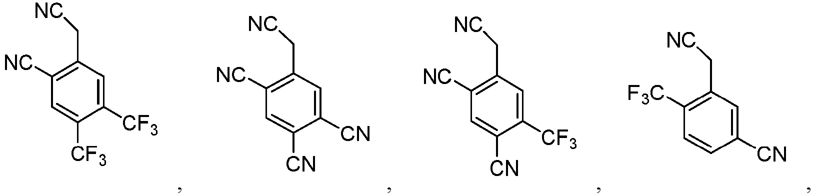

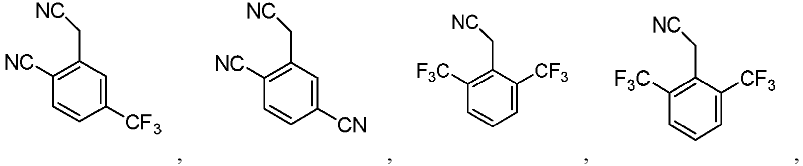

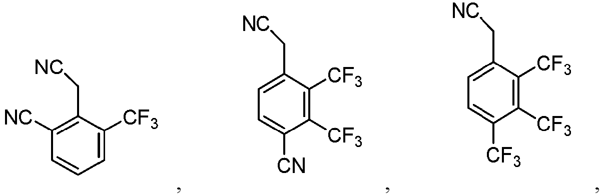

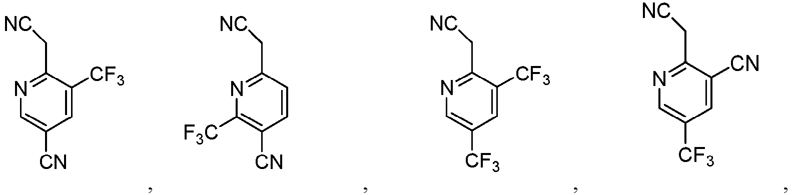

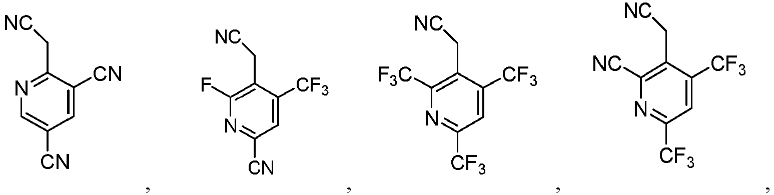

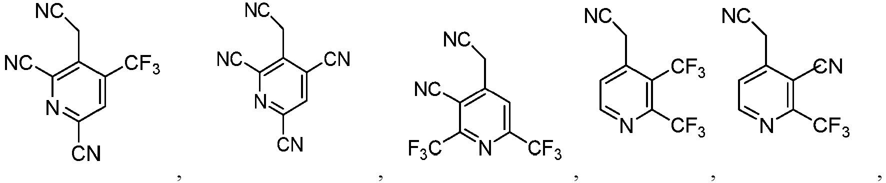

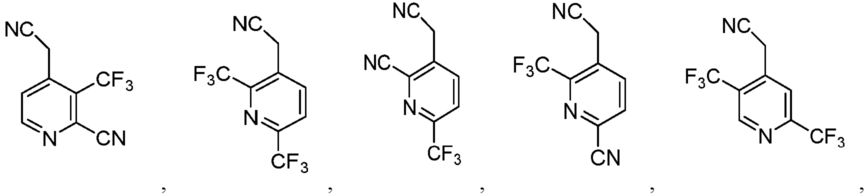

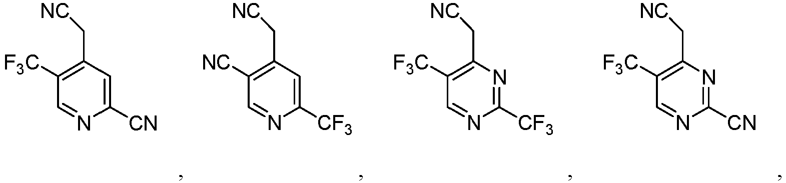

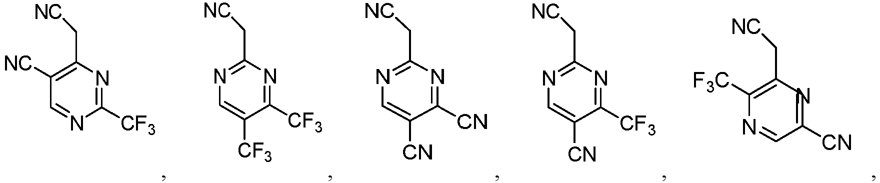

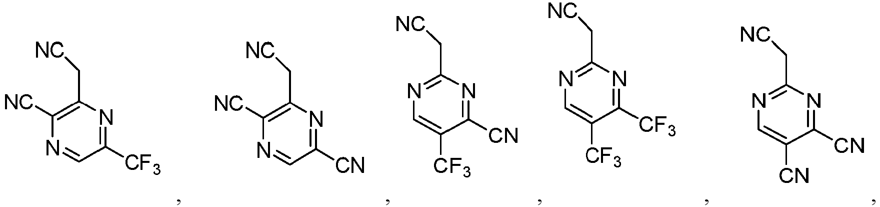

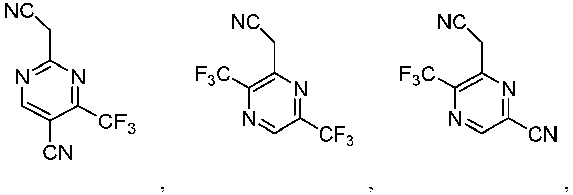

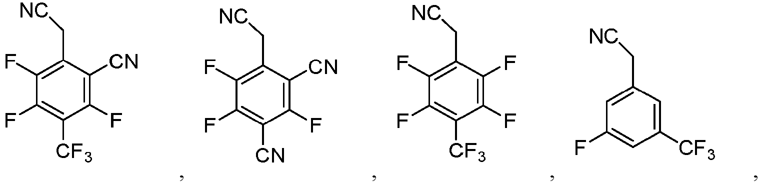

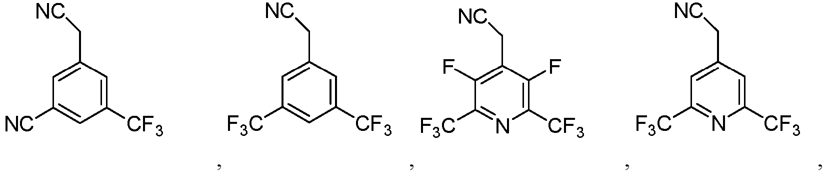

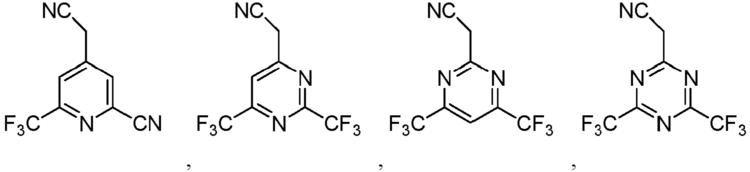

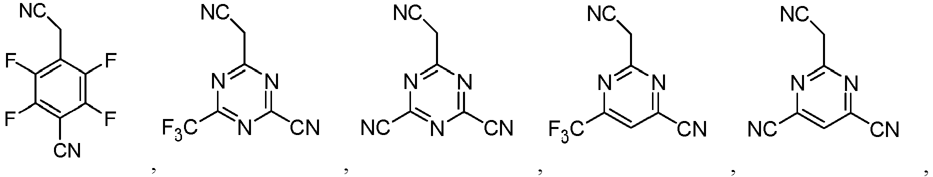

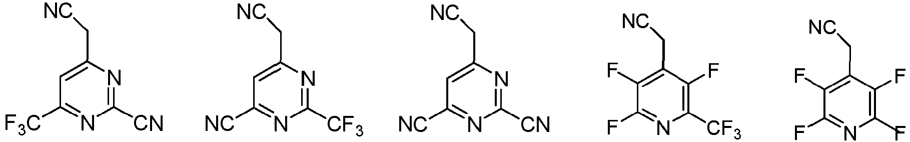

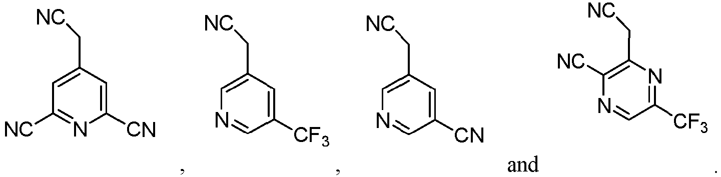

According to one embodiment of the present invention, formula (II) is selected from the group comprising the following moieties:

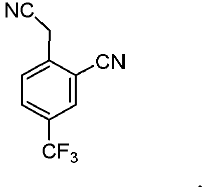

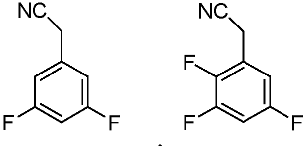

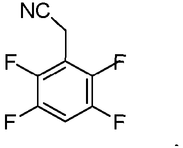

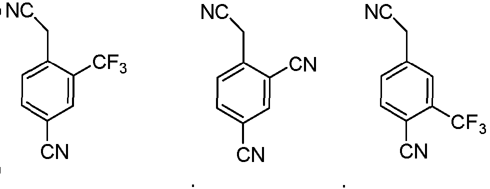

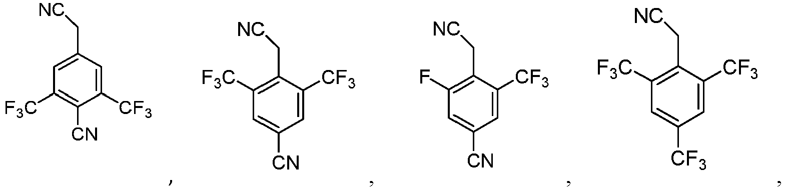

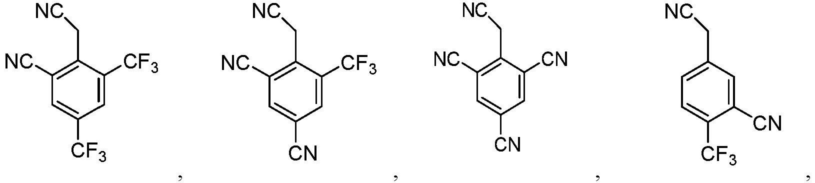

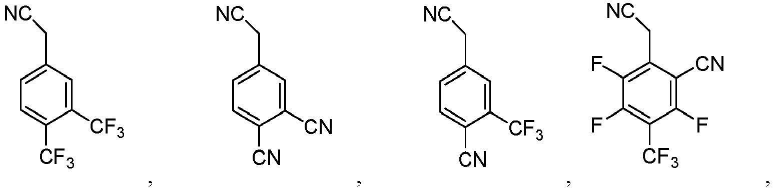

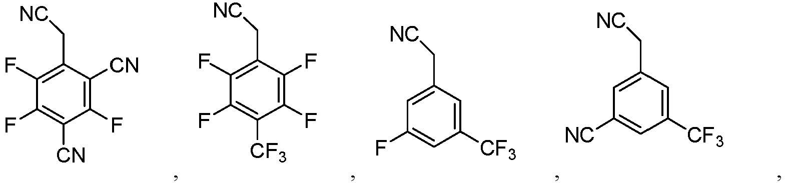

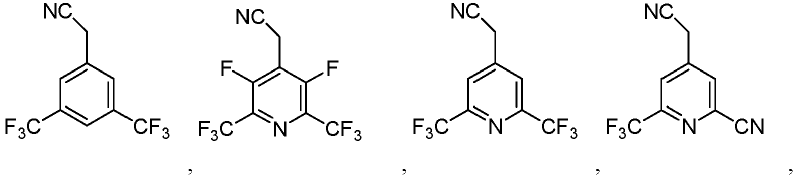

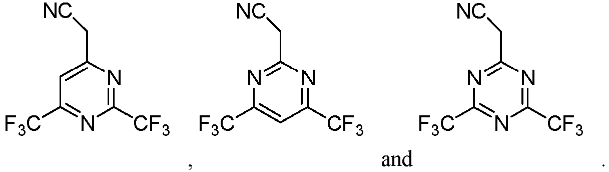

According to one embodiment of the present invention, formula (III) is selected from the group comprising the following moieties:

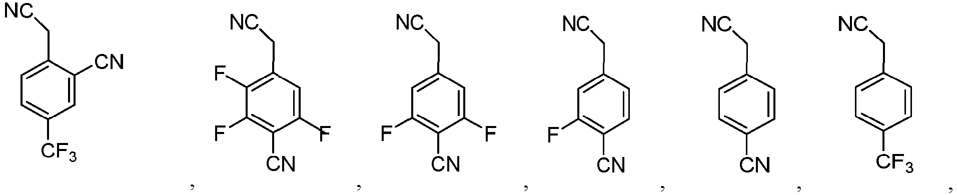

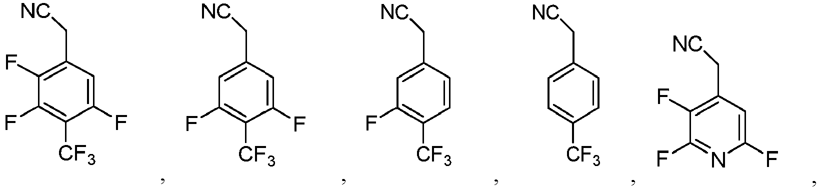

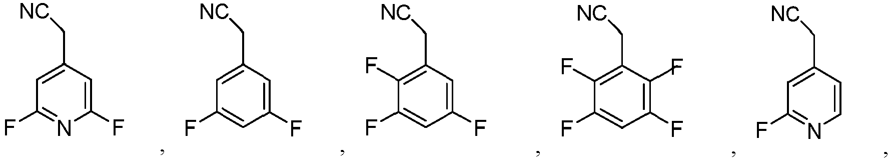

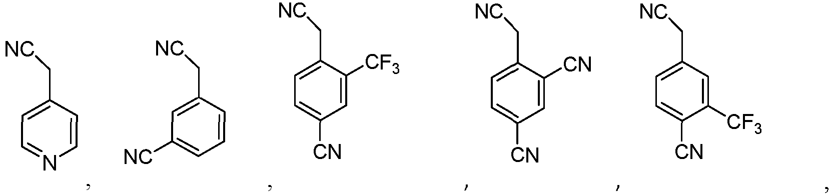

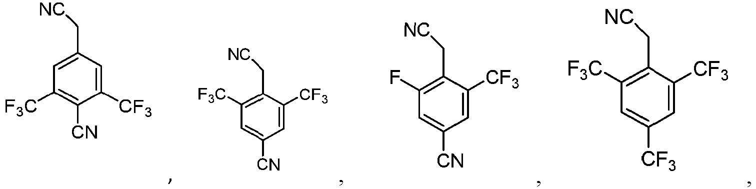

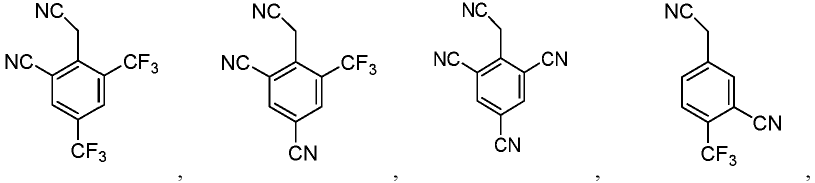

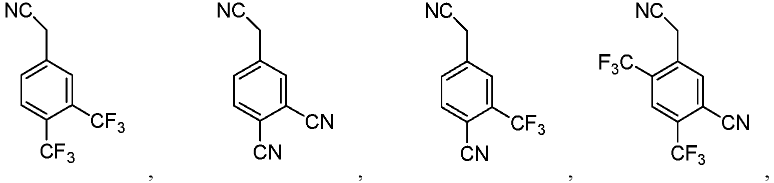

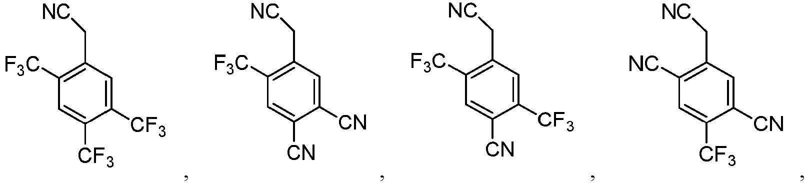

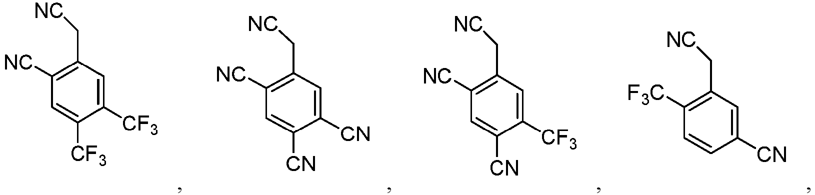

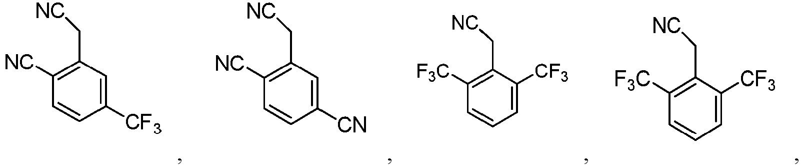

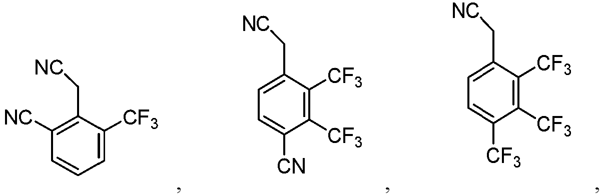

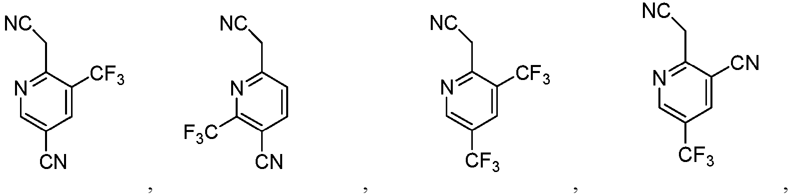

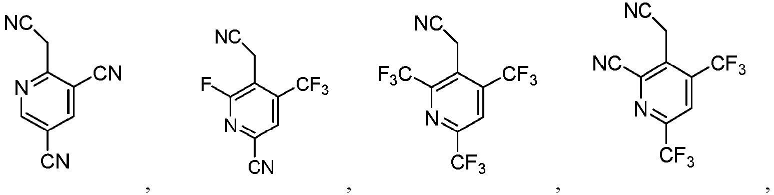

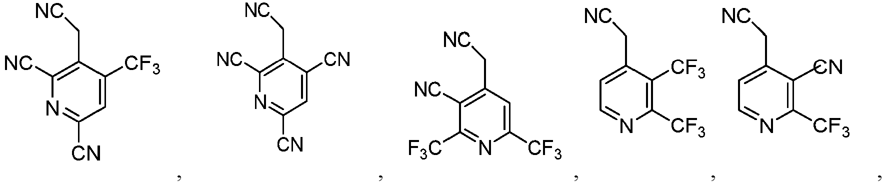

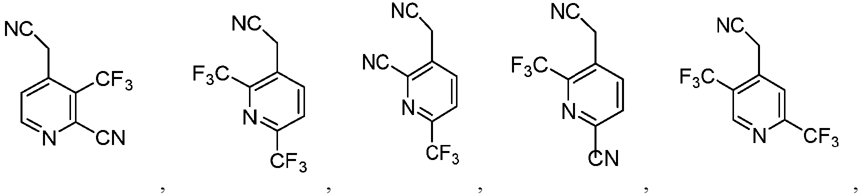

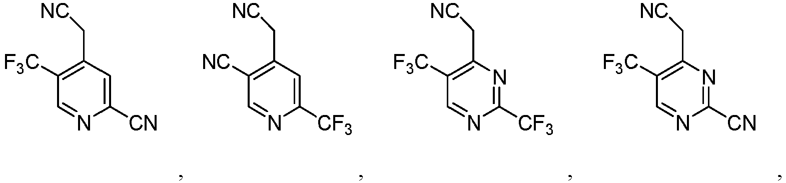

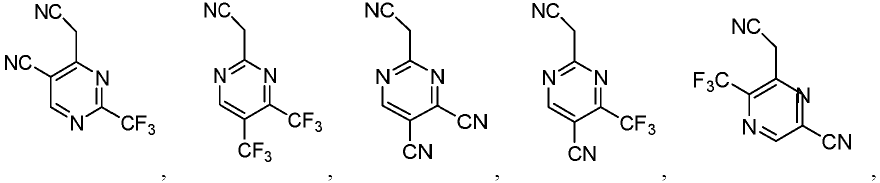

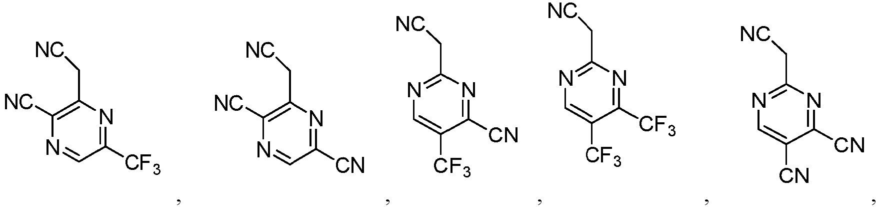

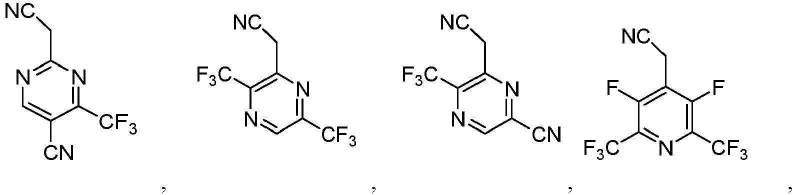

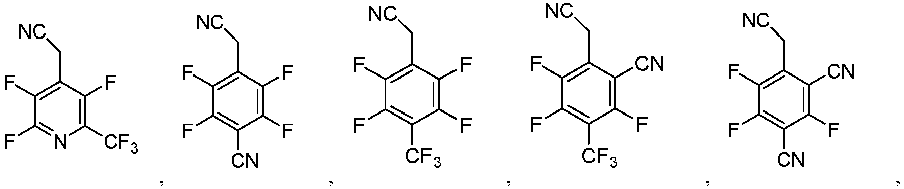

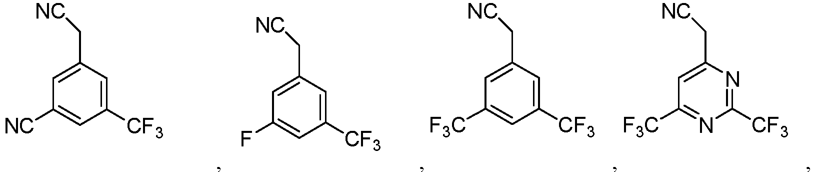

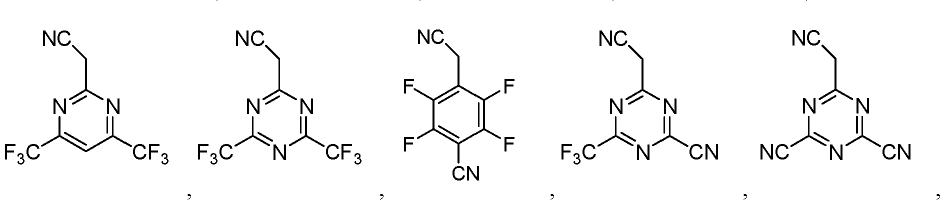

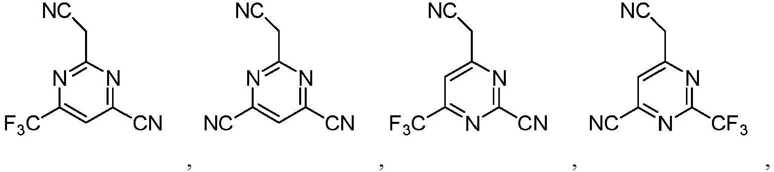

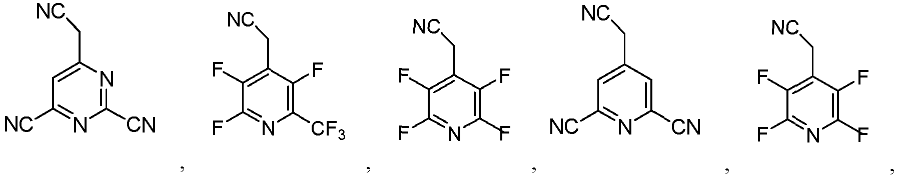

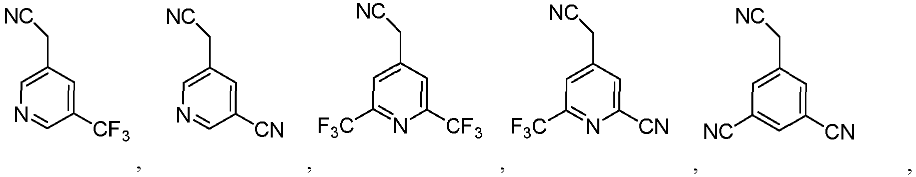

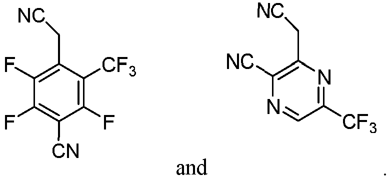

According to one embodiment of the present invention, formula (III) is selected from the group comprising the following moieties:

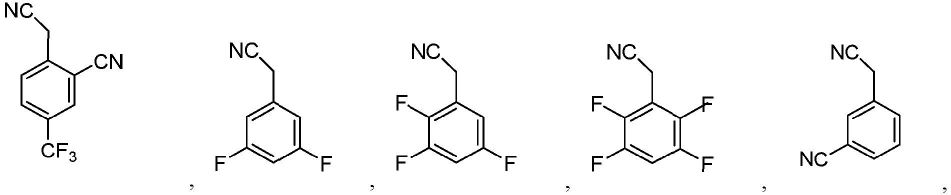

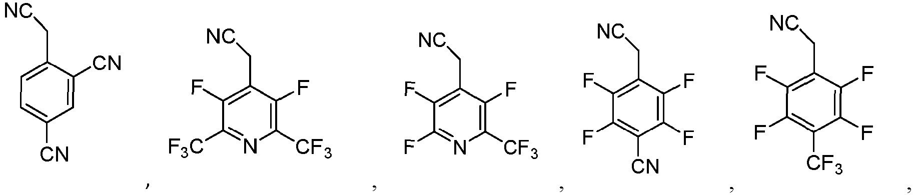

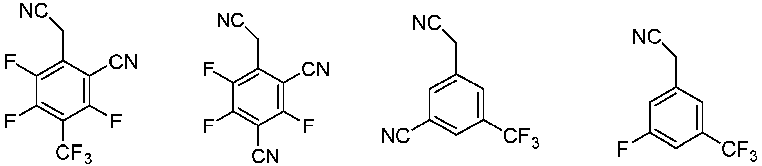

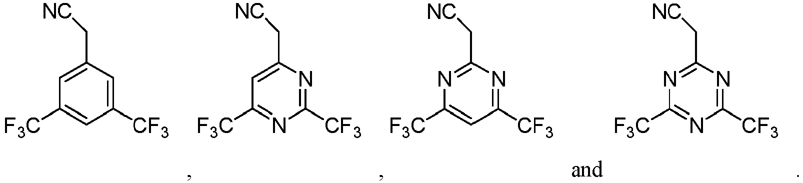

According to one embodiment of the present invention, formula (III) is selected from the group comprising the following moieties:

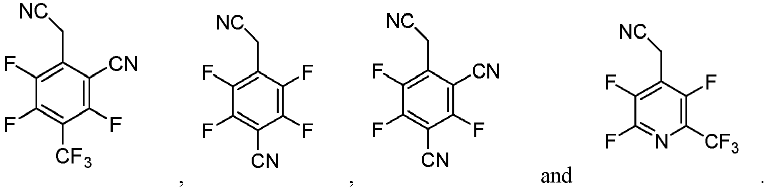

According to one embodiment of the present invention, the LUMO level of compound of formula (I) is selected in the range of ≤ -4.3 eV and ≥ -5.6 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, preferably in the range of ≤ -4.35 eV and ≥ -5.4 eV and most preferred in the range of ≤ -4.35 eV and ≥ -5.15 eV.

According to one embodiment of the present invention, the hole injection layer comprises a compound selected from A¹ to A4 and A8 to A37, Compounds A5 to A7 are reference compounds not forming part of the present invention:

| | A¹ | A² | A³ |
|---|---|---|---|
| A1 | 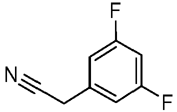 | 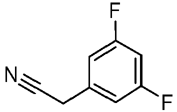 | 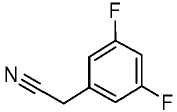 |
| A2 | 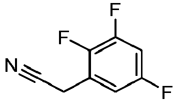 | 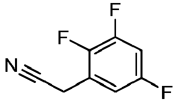 | 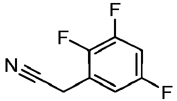 |
| A3 | 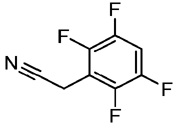 | 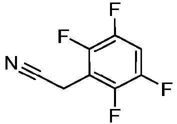 | 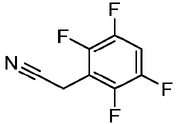 |
| A4 | 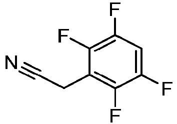 | 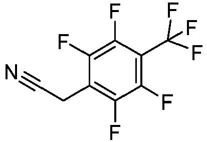 | 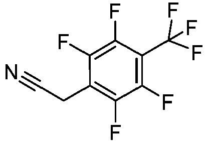 |
| A5 | 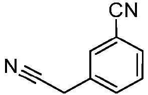 | 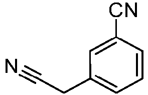 | 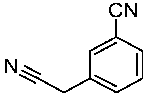 |
| A6 | 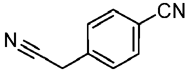 | 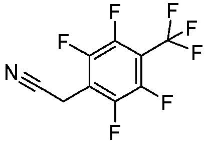 | 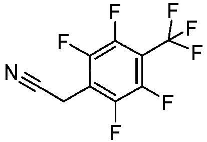 |
| A7 | 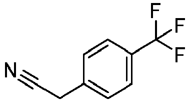 | 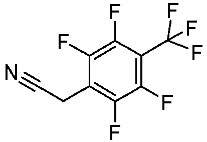 | 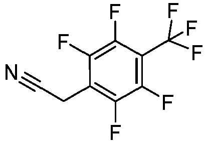 |
| A8 | 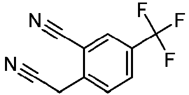 | 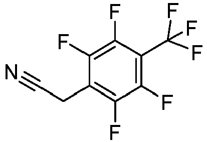 | 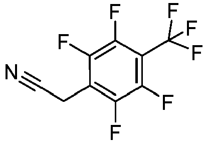 |
| A9 | 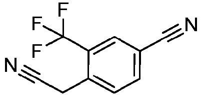 | 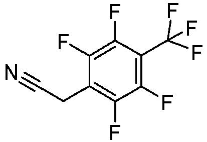 | 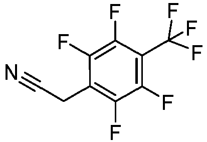 |
| A10 | 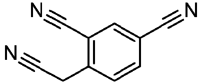 | 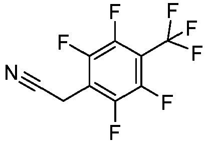 | 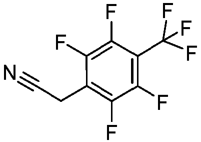 |
| A11 | 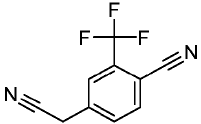 | 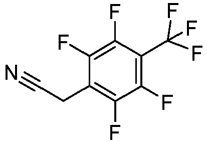 | 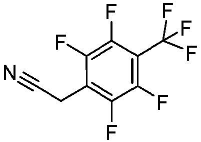 |
| A12 | 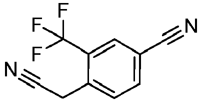 | 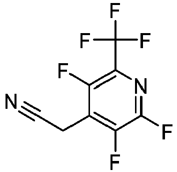 | 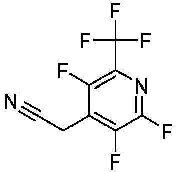 |
| A13 | 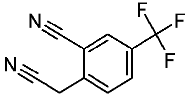 | 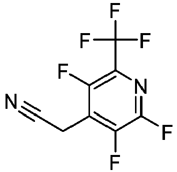 | 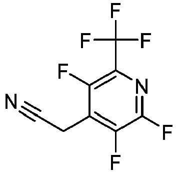 |
| A14 | 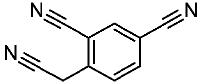 | 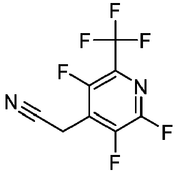 | 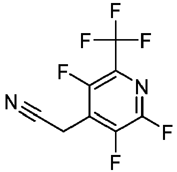 |
| A15 | 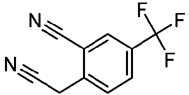 | 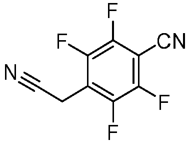 | 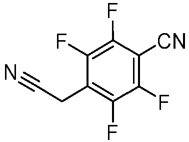 |
| A16 | 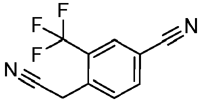 | 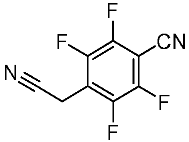 | 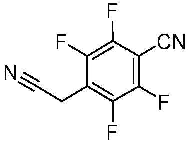 |
| A17 | 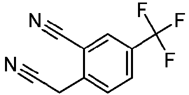 | 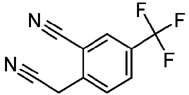 | 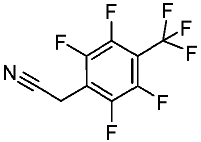 |
| A18 | 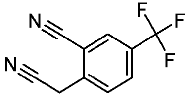 | 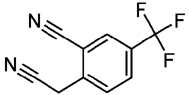 | 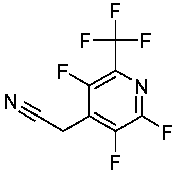 |
| A19 | 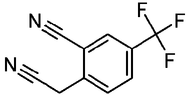 | 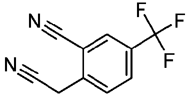 | 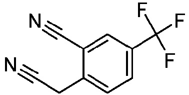 |
| A20 | 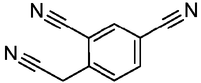 | 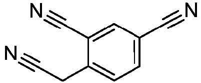 | 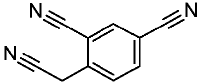 |
| A21 | 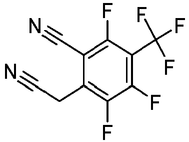 | 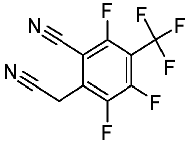 | 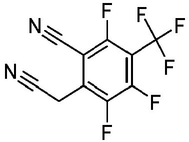 |
| A22 | 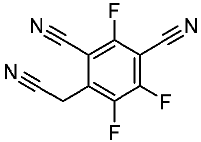 | 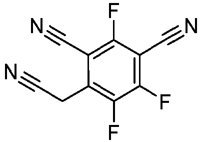 | 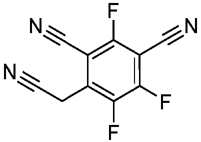 |
| A23 | 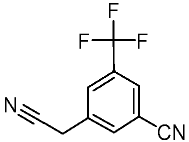 | 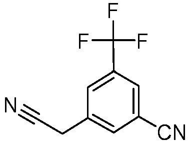 | 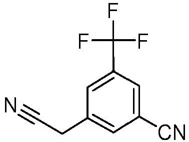 |
| A24 | 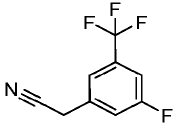 | 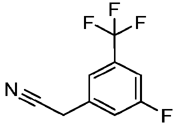 | 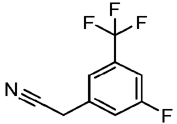 |
| A25 | 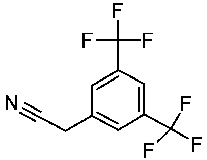 | 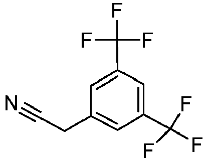 | 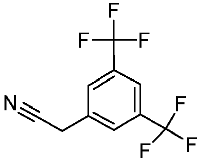 |
| A26 | 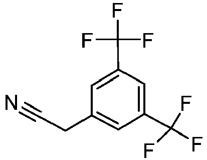 | 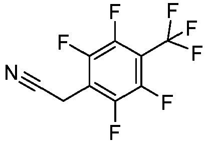 | 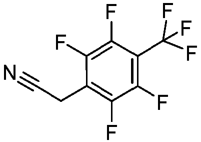 |
| A27 | 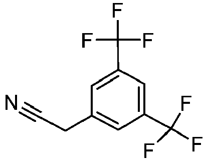 | 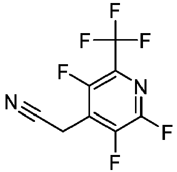 | 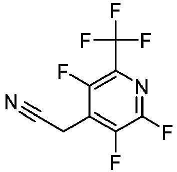 |
| A28 | 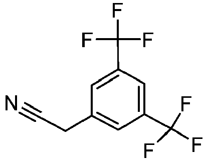 | 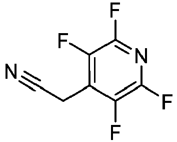 | 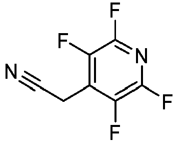 |
| A29 | 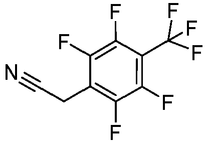 | 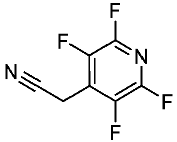 | 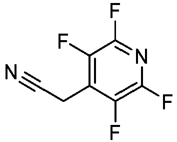 |
| A30 | 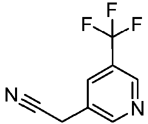 | 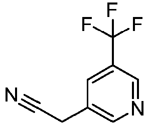 | 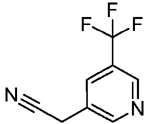 |
| A31 | 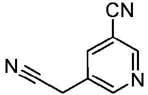 | 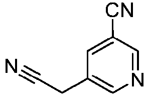 | 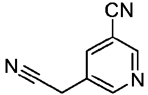 |
| A32 | 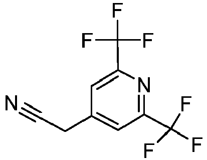 | 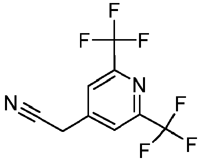 | 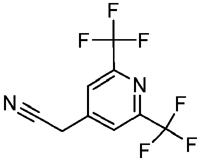 |
| A33 | 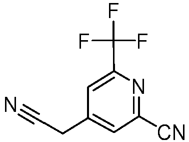 | 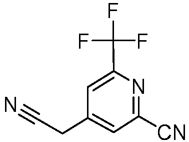 | 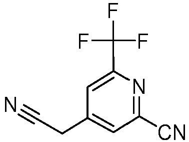 |
| A34 | 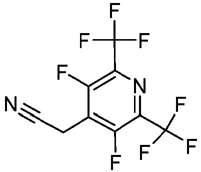 | 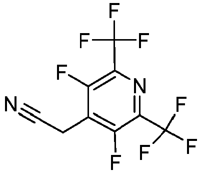 | 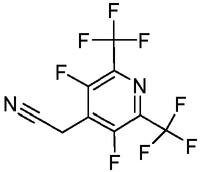 |
| A35 | 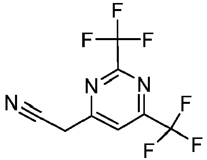 | 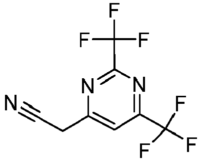 | 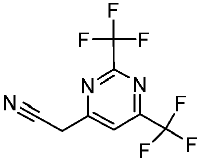 |
| A36 | 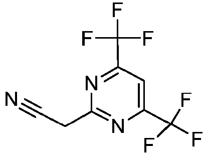 | 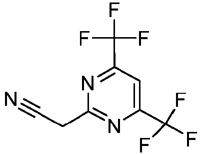 | 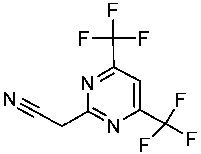 |
| A37 | 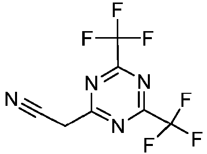 | 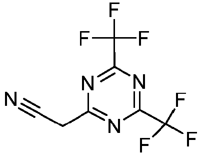 | 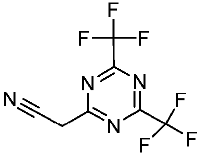 |

According to one embodiment of the present invention the hole injection layer and/or the compound of formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

The present invention furthermore relates to a compound of formula (I) of claim 1, wherein formula (III) is selected from the group consisting of

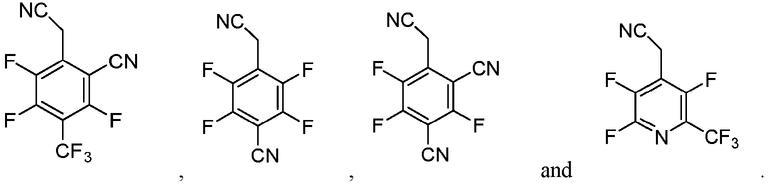

According to an embodiment of the present invention, the hole injection layer comprises a substantially covalent matrix compound.

### Substantially covalent matrix compound

According to one embodiment the substantially covalent matrix compound may be selected from at least one organic compound, which may consists substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compound of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

### Compound of formula (VI) or a compound of formula (VII)

According to another aspect of the present invention, the substantially covalent matrix compound may comprise at least one arylamine compound, diarylamine compound, triarylamine compound, a compound of formula (VI) or a compound of formula (VII): 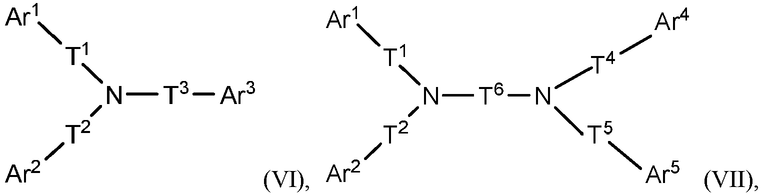 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar^{!}, Ar², Ar³, Ar⁴ and Ar⁵ may be independently selected from D1 to D16: 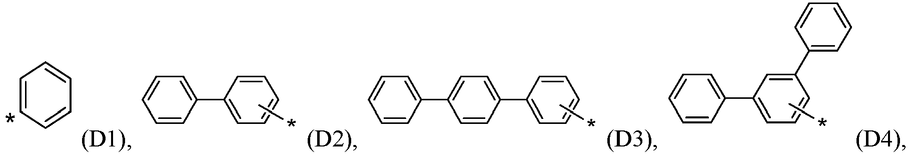 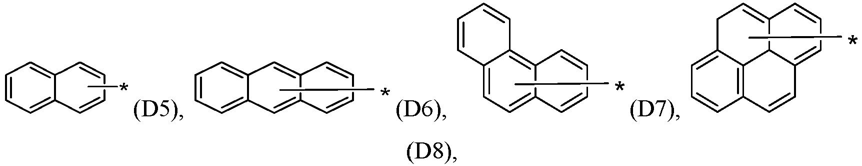 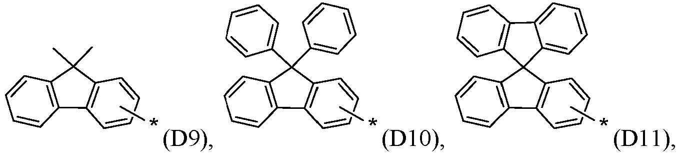 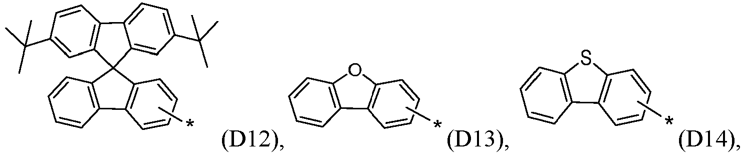 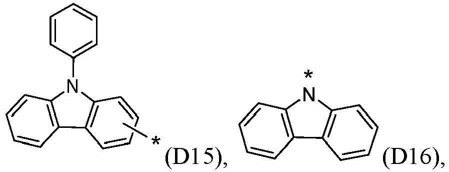 wherein the asterix "*" denotes the binding position.

According to an embodiment, wherein Ar¹, Ar², Ar³, Ar⁴ and Ar⁵ may be independently selected from D1 to D15; alternatively selected from D1 to D10 and D13 to D15.

According to an embodiment, wherein Ar¹, Ar², Ar³, Ar⁴ and Ar⁵ may be independently selected from the group consisting of D1, D2, D5, D7, D9, D10, D13 to D16.

The rate onset temperature may be in a range particularly suited to mass production, when Ar¹, Ar², Ar³, Ar⁴ and Ar⁵ are selected in this range.

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the invention, the substantially covalent matrix compound comprises a compound selected from F1 to F18:

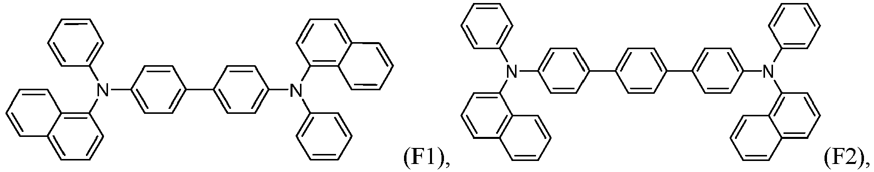

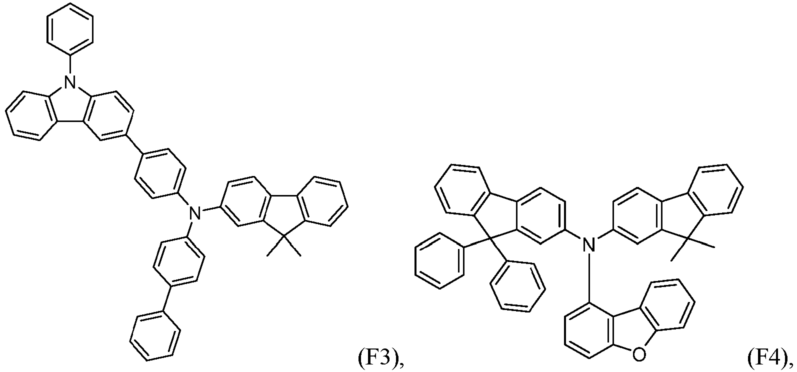

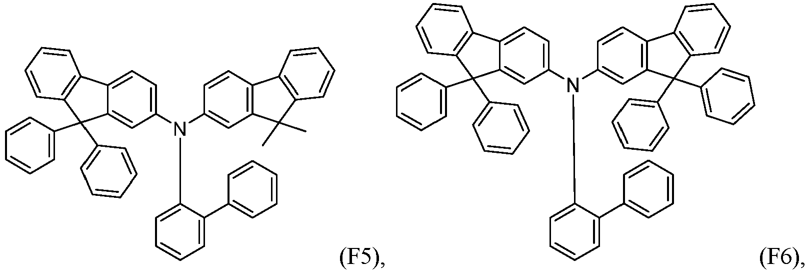

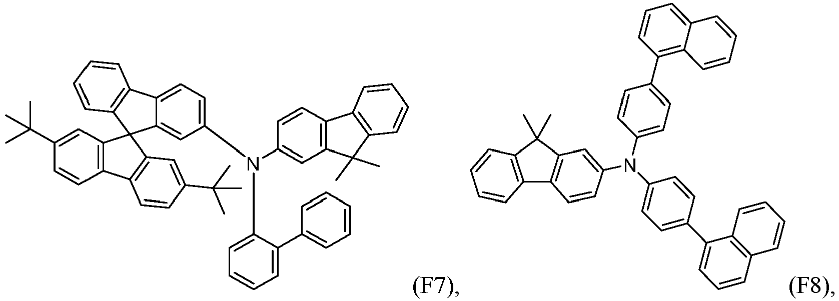

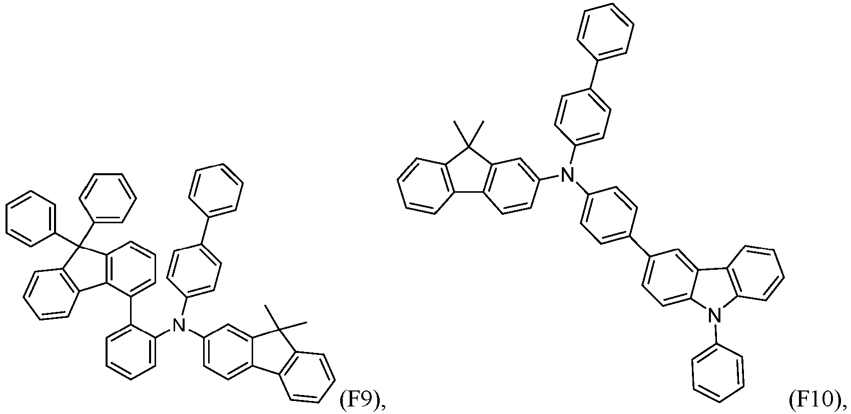

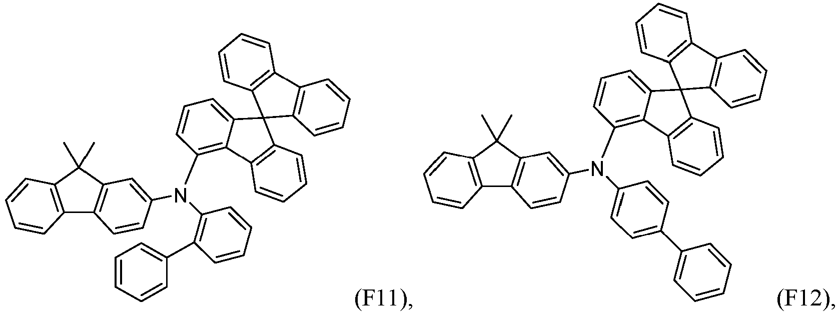

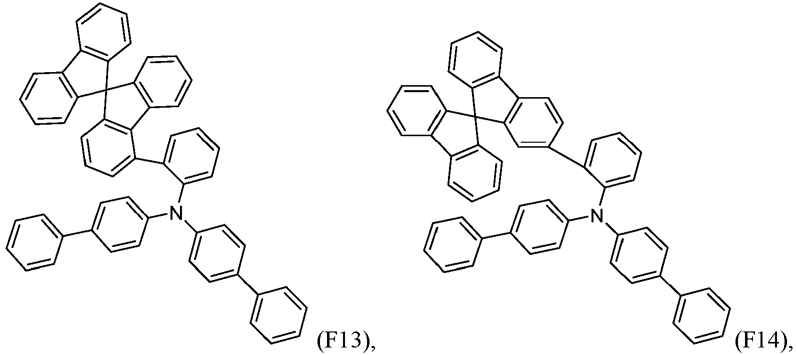

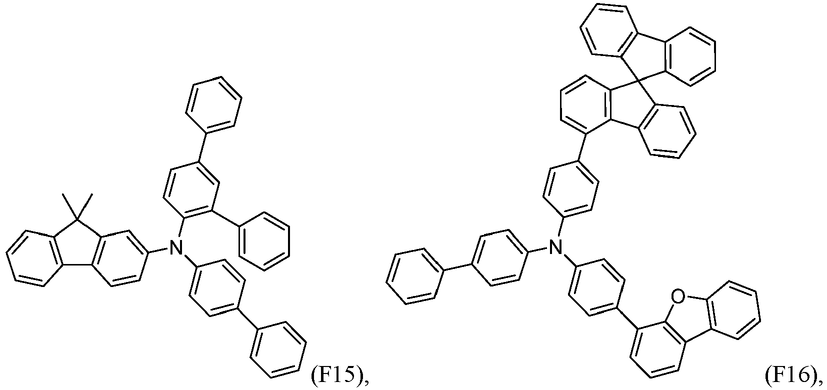

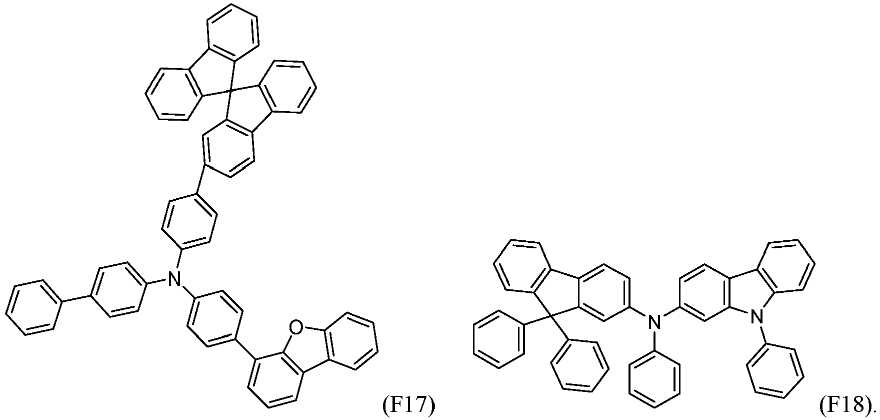

According to one embodiment of the invention the electronic organic device is an electroluminescent device, preferably an organic light emitting diode.

The present invention furthermore relates to a display device comprising an organic electronic device according to the present invention.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Hole transport layer

According to one embodiment of the present invention, the whereby the organic electronic device comprises a hole transport layer, wherein the hole transport layer is arranged between the hole injection layer and the at least one first emission layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise a substantially covalent matrix compound as described above.

According to one embodiment of the present invention, the hole transport layer may comprise a compound of formula (VI) or (VII) as described above.

According to one embodiment of the present invention, the hole injection layer and the hole transport layer comprises the same substantially covalent matrix compound as described above.

According to one embodiment of the present invention, the hole injection layer and the hole transport layer comprises the same compound of formula (VI) or (VII) as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; a hole injection layer comprising a compound of formula (I), a hole transport layer, an emission layer, an electron transport layer and a cathode electrode.

According to another aspect of the present invention, there is provided an OLED comprising: a substrate; an anode electrode formed on the substrate; a hole injection layer comprising a compound of formula (I), a hole transport layer, an electron blocking layer, an emission layer, a hole blocking layer, an electron transport layer and a cathode electrode.

According to another aspect of the present invention, there is provided an OLED comprising: a substrate; an anode electrode formed on the substrate; a hole injection layer comprising a compound of formula (I), a hole transport layer, an electron blocking layer, an emission layer, a hole blocking layer, an electron transport layer, an electron injection layer, and a cathode electrode.

According to various embodiments of the present invention, there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

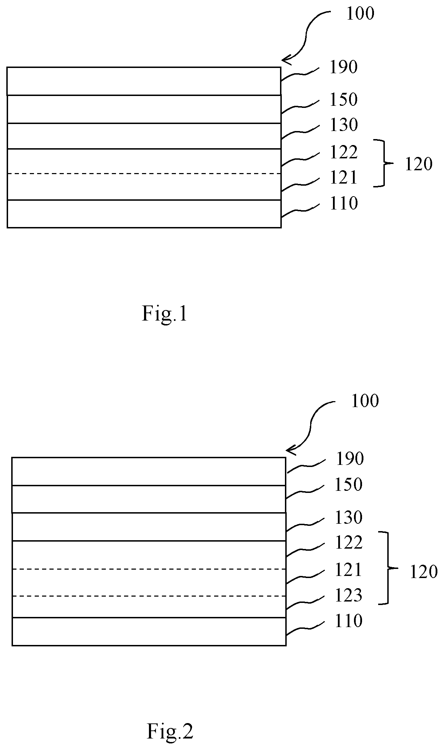

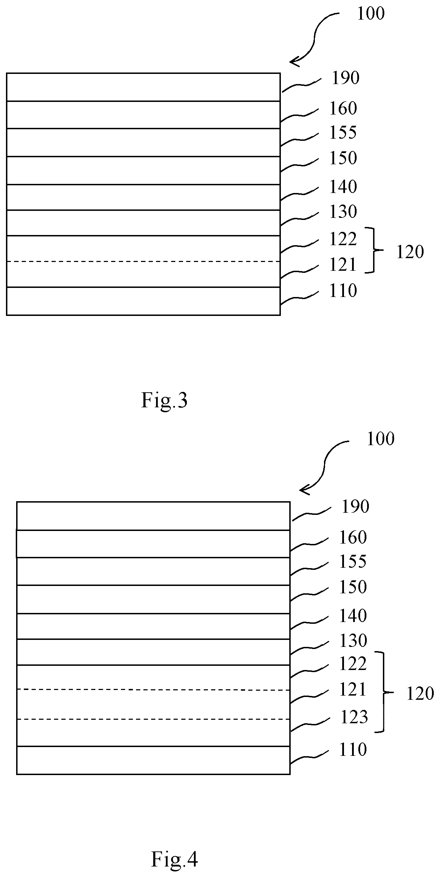

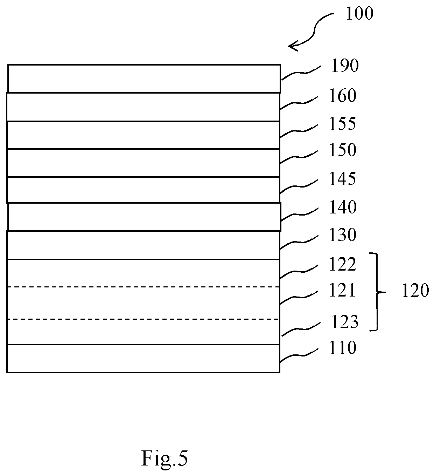

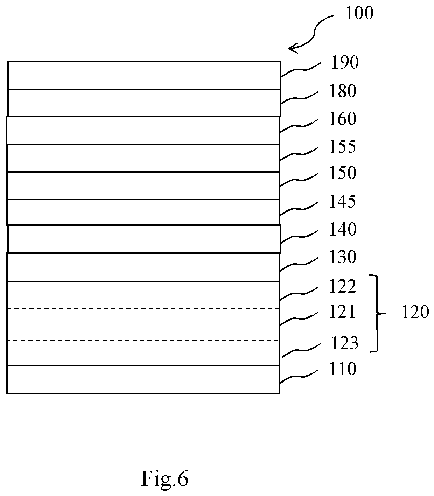

For example, the OLED according to Fig. 2 may be formed by a process, wherein on a substrate (110), an anode (120), a hole injection layer (130) which may comprise compound of formula (I), a hole transport layer (140), an electron blocking layer (145), an emission layer (150), a hole blocking layer (155), an electron transport layer (160), an electron injection layer (180) and the cathode electrode (190) are subsequently formed in that order.

### Organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of formula (I) according to the invention, and
- a second deposition source to release the substantially covalent matrix compound; the method comprising the steps of forming the hole injection layer; whereby for an organic light-emitting diode (OLED):
- the hole injection layer is formed by releasing the compound of formula (I) according to the invention from the first deposition source and the substantially covalent matrix compound from the second deposition source.

According to various embodiments of the present invention, the method may further include forming on the anode electrode, at least one layer selected from the group consisting of forming a hole transport layer or forming a hole blocking layer, and an emission layer between the anode electrode and the first electron transport layer.

According to various embodiments of the present invention, the method may further include the steps for forming an organic light-emitting diode (OLED), wherein
- on a substrate an anode electrode is formed,
- on the anode electrode a hole injection layer comprising a compound of formula (I) is formed,
- on the hole injection layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode electrode is formed,
- optional a hole blocking layer is formed in that order between the first anode electrode and the emission layer,
- optional an electron injection layer is formed between the electron transport layer and the cathode electrode.

According to various embodiments, the OLED may have the following layer structure, wherein the layers having the following order:
anode, hole injection layer comprising a compound of formula (I) according to the invention, first hole transport layer, second hole transport layer, emission layer, optional hole blocking layer, electron transport layer, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Embodiments

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

According to an exemplary embodiment of the present invention, the organic electronic device includes a substrate, an anode layer that comprises a first anode sub-layer and a second anode sub-layer and a hole injection layer (HIL). The HIL is disposed on the anode layer. Onto the HIL, a first emission layer (EML), and a cathode layer are disposed.

According to an exemplary embodiment of the present invention, the organic electronic device includes a substrate, an anode layer hat comprises a first anode sub-layer, a second anode sub-layer and a third anode sub-layer, and a hole injection layer (HIL). The HIL is disposed on the anode layer comprising a first anode sub-layer, a second anode sub-layer and a third anode sub-layer. Onto the HIL, a first emission layer (EML), and a cathode layer are disposed.

According to an exemplary embodiment of the present invention, the organic electronic device includes a substrate, an anode layer that comprises a first anode sub-layer and a second anode sub-layer, and a hole injection layer (HIL). The HIL is disposed on the anode layer. Onto the HIL, an hole transport layer (HTL), a first emission layer (EML), a hole blocking layer (BL), an electron transport layer (ETL), and a cathode layer are disposed.

According to an exemplary embodiment of the present invention, the organic electronic device includes a substrate, an anode layer that comprises a first anode sub-layer, a second anode sub-layer and a third anode sub-layer, and a hole injection layer (HIL). The HIL is disposed on the anode layer. Onto the HIL, an hole transport layer (HTL), a first emission layer (EML), a hole blocking layer (HBL), an electron transport layer (ETL), and a cathode layer are disposed.

According to an exemplary embodiment of the present invention, the organic electronic device includes a substrate, an anode layer that comprises a first anode sub-layer and a second anode sub-layer and a hole injection layer (HIL). The HIL is disposed on the anode layer. Onto the HIL, a hole transport layer (HTL), an electron blocking layer (EBL), a first emission layer (EML), a hole blocking layer (HBL), an electron transport layer (ETL), and a cathode layer are disposed.

According to an exemplary embodiment of the present invention, the organic electronic device includes a substrate, an anode layer that comprises a first anode sub-layer, a second anode sub-layer and a third anode sub-layer, and a hole injection layer (HIL). The HIL is disposed on the anode layer. Onto the HIL, a hole transport layer (HTL) , an electron blocking layer (EBL), a first emission layer (EML), a hole blocking layer (HBL), an electron transport layer (ETL), an electron injection layer (EIL) and a cathode layer are disposed.

A capping and/or a sealing layer may further be formed on the cathode layer, in order to seal the organic electronic device. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Detailed description

The invention is furthermore illustrated by the following examples which are illustrative only and non-binding.

Compounds of formula (I) may be prepared as described in EP2180029A1 and WO2016097017A1.

### Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

### General procedure for fabrication of OLEDs

For Examples 1 to 16 and comparative example 2 in Table 2, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment, see Table 2, to prepare the anode layer. The plasma treatment was performed in nitrogen atmosphere or in an atmosphere comprising 97.6 vol.-% nitrogen and 2.4 vol.-% oxygen.

Then, compound of formula F3 as matrix compound and compound of formula (I) were co-deposited in vacuum on the anode layer, to form a hole injection layer (HIL) having a thickness of 10 nm. The percentage compound of formula (I) in the HIL can be seen in Table 2.

Then, compound of formula F3 was vacuum deposited on the HIL, to form a HTL having a thickness of 123 nm.

Then N-([1,1'-biphenyl]-4-yl)-9,9-diphenyl-N-(4-(triphenylsilyl)phenyl)-9H-fluoren-2-amine (CAS 1613079-70-1) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue emitter dopant were deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer having a thickness of 31 nm was formed on the hole blocking layer by depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% of LiQ.

Then Ag:Mg (90:10 vol.-%) was evaporated at a rate of 0.01 to 1 Å/s at 10⁻⁷ mbar to form a cathode layer with a thickness of 13 nm on the electron transporting layer.

Then, compound of formula F3 was deposited on the cathode layer to form a capping layer with a thickness of 75 nm.

### Comparative example 1

For comparative example 1 in Table 3, a 15Ω /cm² glass substrate with 90 nm ITO (available from Corning Co.) was cut to a size of 50 mm x 50 mm x 0.7 mm, ultrasonically washed with water for 60 minutes and then with isopropanol for 20 minutes. The liquid film was removed in a nitrogen stream, followed by plasma treatment in nitrogen atmosphere at a power of 100 W for 75 seconds to prepare the anode layer.

Then, 92 wt.-% compound of formula F3 and 8 wt.-% compound A8 were co-deposited in vacuum on the anode layer, to form a hole injection layer (HIL) having a thickness of 10 nm.

Then, compound of formula F3 was vacuum deposited on the HIL, to form a HTL having a thickness of 123 nm.

Then, the EBL, EML, HBL and ETL are deposited in this order on the HTL, as described for example 1 above.

Then Yb was evaporated at a rate of 0.01 to 1 Å/s at 10⁻⁷ mbar to form an electron injection layer with a thickness of 2 nm on the electron transporting layer.

Then A¹ was evaporated at a rate of 0.01 to 1 Å/s at 10⁻⁷ mbar to form a cathode layer with a thickness of 100 nm on the electron injection layer.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing an operating voltage U in V and measuring the current in mA flowing through the device under test. The voltage applied to the device is varied in steps of 0.1V in the range between 0 V and 10 V.

### Technical Effect of the invention

In Table 1 are shown LUMO levels for Examples A1 to A4, A8 to A37, Reference Examples A5 to A7 not forming part of the present invention, and comparative example 1 (=C1). As comparative compound (referred to as C1) a compound with A¹ to A³ = Phenyl and R' = CN was used.

LUMO levels were calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

**Table 1: Calculated LUMO level of compounds of formula (I)**

| | A¹ | A² | A³ | LUMO [eV] |
|---|---|---|---|---|
| C1 | 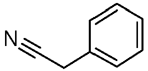 | 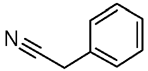 | 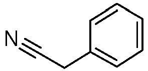 | -3.81 |
| A1 | 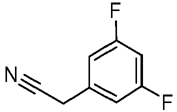 | 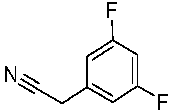 | 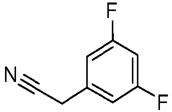 | -4.34 |
| A2 | 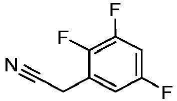 | 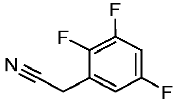 | 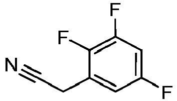 | -4.39 |
| A3 | 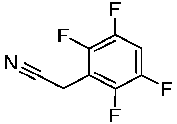 | 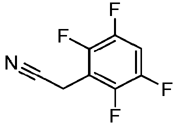 | 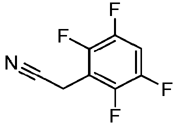 | -4.44 |
| A4 | 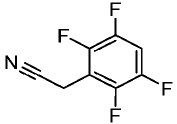 | 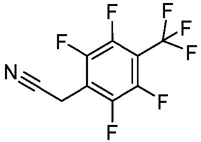 | 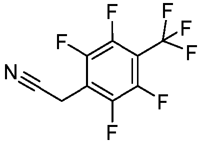 | -4.75 |
| A5 | 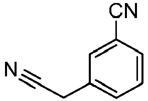 | 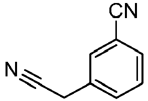 | 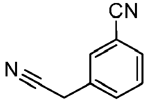 | -4.58 |
| A6 | 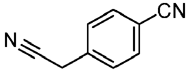 | 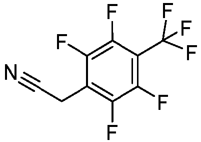 | 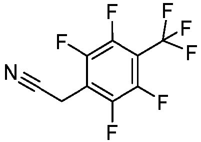 | -4.85 |
| A7 | 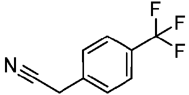 | 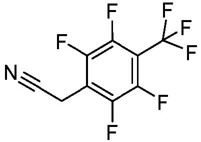 | 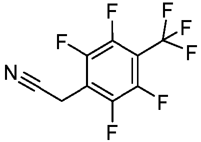 | -4.75 |
| A8 | 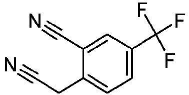 | 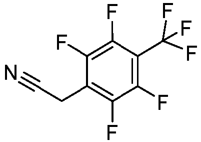 | 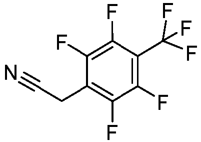 | -4.82 |
| A9 | 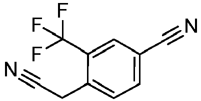 | 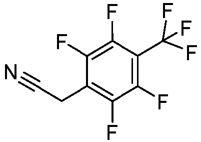 | 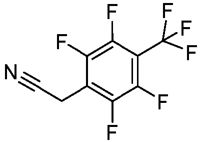 | -4.83 |
| A10 | 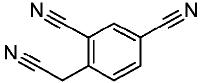 | 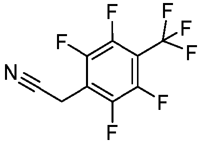 | 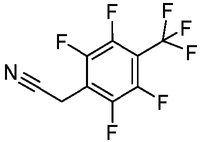 | -4.92 |
| A11 | 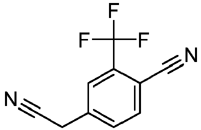 | 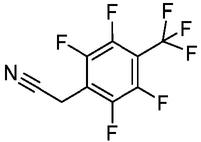 | 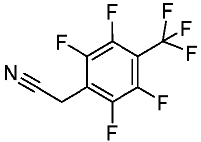 | -4.95 |
| A12 | 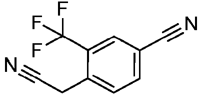 | 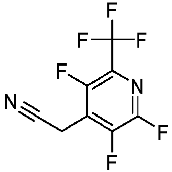 | 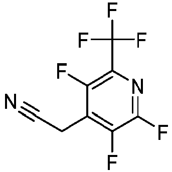 | -4.99 |
| A13 | 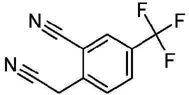 | 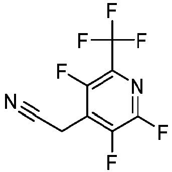 | 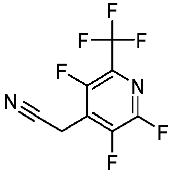 | -5.12 |
| A14 | 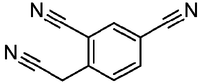 | 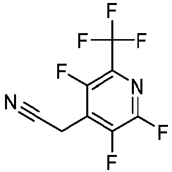 | 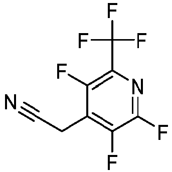 | -5.16 |
| A15 | 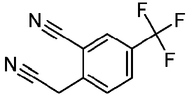 | 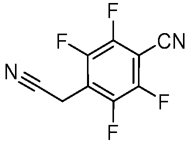 | 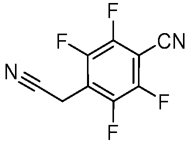 | -5.09 |
| A16 | 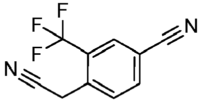 | 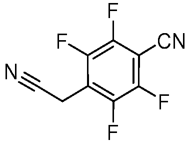 | 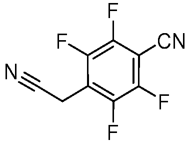 | -5.07 |
| A17 | 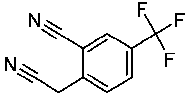 | 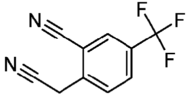 | 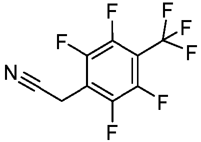 | -4.73 |
| A18 | 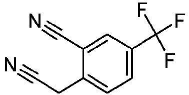 | 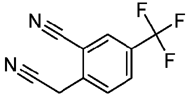 | 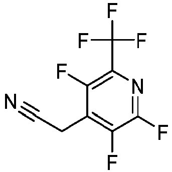 | -4.91 |
| A19 | 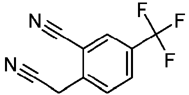 | 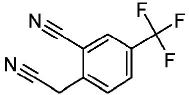 | 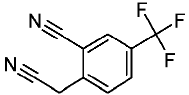 | -4.71 |
| A20 | 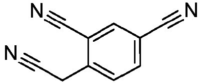 | 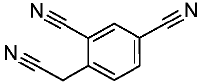 | 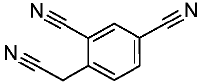 | -5.07 |
| A21 | 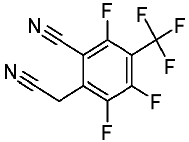 | 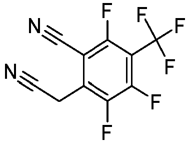 | 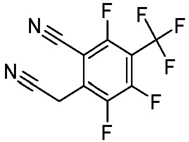 | -4.71 |
| A22 | 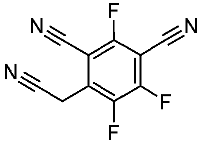 | 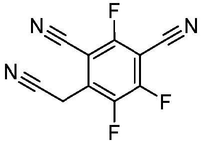 | 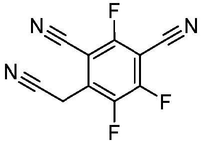 | -5.50 |
| A23 | 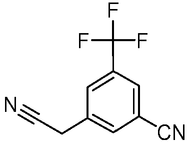 | 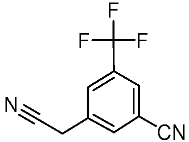 | 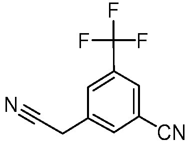 | -4.95 |
| A24 | 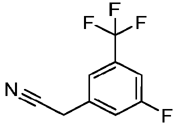 | 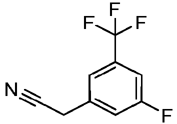 | 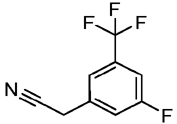 | -4.52 |
| A25 | 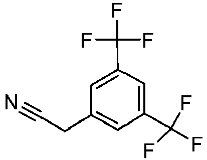 | 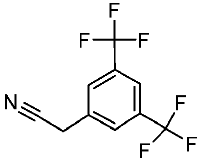 | 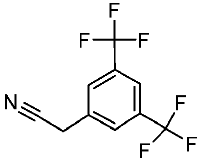 | -4.92 |
| A26 | 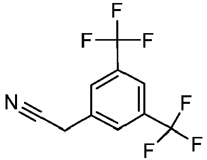 | 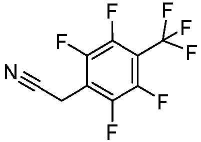 | 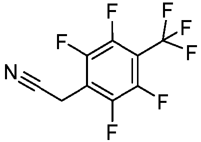 | -4.82 |
| A27 | 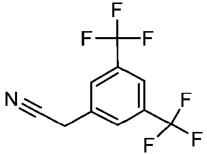 | 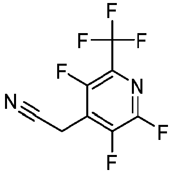 | 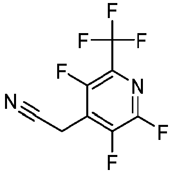 | -5.07 |
| A28 | 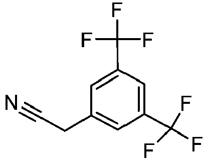 | 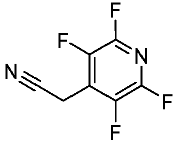 | 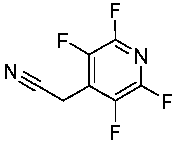 | -4.93 |
| A29 | 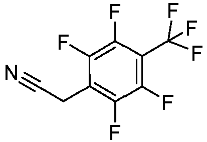 | 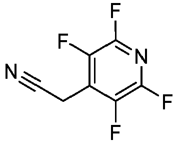 | 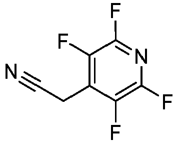 | -5.03 |
| A30 | 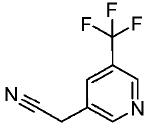 | 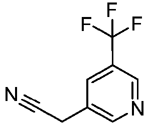 | 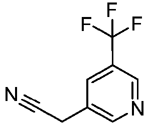 | -4.66 |
| A31 | 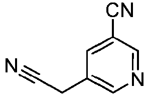 | 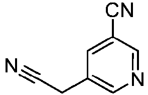 | 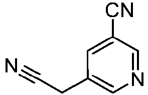 | -4.95 |
| A32 | 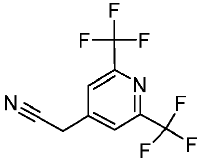 | 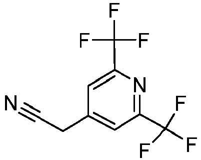 | 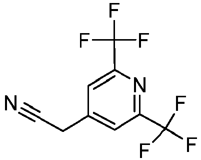 | -5.33 |
| A33 | 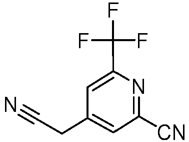 | 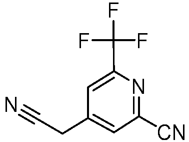 | 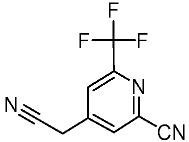 | -5.58 |
| A34 | 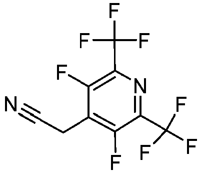 | 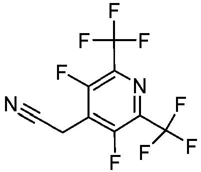 | 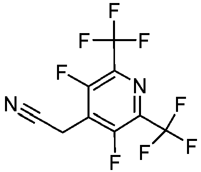 | -5.36 |
| A35 | 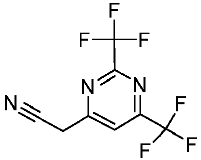 | 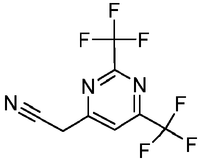 | 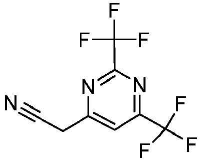 | -5.50 |
| A36 | 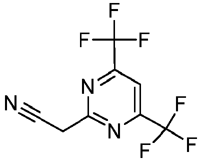 | 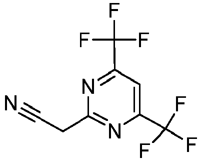 | 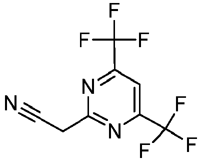 | -4.96 |
| A37 | 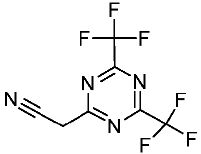 | 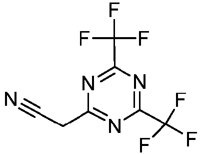 | 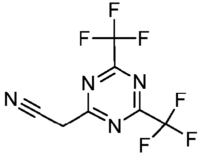 | -5.61 |

**Table 2 shows the setup and the operating voltage of one device according to comparative examples 1 and 2 and to examples 1 to 16 according to the invention:**

| | Anode | Compound of formula (I) | Percentage compound of formula (I) [vol.-%] | U at 50 mA/cm² [V] |
|---|---|---|---|---|
| Comparative example 1 | ITO | A8 | 8 | 4.23 |
| Comparative example 2 | ITO/Ag/ITO | C1 | 8 | 4.9 |
| Example 1 | ITO/Ag/ITO | A8 | 8 | 3.84 |
| Example 2 | ITO/Ag/ITO | A8 | 12 | 3.83 |
| Example 3 | ITO/Ag/ITO | A8 | 15 | 3.82 |
| Example 4 | ITO/Ag/ITO | A10 | 8 | 3.83 |
| Example 5 | ITO/Ag/ITO | A26 | 8 | 3.85 |
| Example 6 | ITO/Ag/ITO | A26 | 15 | 3.83 |
| Example 7 | ITO/Ag/ITO | A4 | 8 | 3.87 |
| Example 8 | ITO/Ag/ITO | A11 | 8 | 3.84 |
| Example 9 | ITO/Ag/ITO | A24 | 8 | 3.86 |
| Example 10 | ITO/Ag/ITO | A14 | 8 | 3.79 |
| Example 11 | ITO/Ag/ITO | A16 | 8 | 3.8 |
| Example 12 | ITO/Ag/ITO | A18 | 8 | 3.82 |
| Example 13 | ITO/Ag/ITO | A20 | 8 | 3.81 |
| Example 14 | ITO/Ag/ITO | A29 | 8 | 3.82 |
| Example 15 | ITO/Ag/ITO | A31 | 8 | 3.83 |
| Example 16 | ITO/Ag/ITO | A36 | 8 | 3.84 |

A low operating voltage U may be beneficial for reduced power consumption and improved battery life, in particular in mobile devices.

The particular combinations of elements and features in the above detailed embodiments are exemplary only; Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims.

## Claims

1. An organic electronic device comprising a substrate, an anode layer, a cathode layer, at least one first emission layer, and a hole injection layer, wherein the hole injection layer is arranged between the first emission layer and the anode layer,
and
whereby the hole injection layer comprises a compound of formula (I) 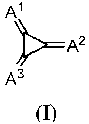
whereby A¹ is selected from formula (II) 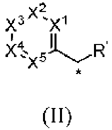
X¹ is selected from CR¹ or N;
X² is selected from CR² or N;
X³ is selected from CR³ or N;
X⁴ is selected from CR⁴ or N;
X⁵ is selected from CR⁵ or N;
R¹, R² , R³, R⁴ and R⁵, if present are independently selected from CN, partially fluorinated or perfluorinated C₁ to Cs alkyl, halogen, Cl, F, D or H, whereby when any of R¹, R², R³, R⁴ and R⁵ is present, then the corresponding X¹, X², X³, X⁴ and X⁵ is not N;
with the proviso that at least two of the following requirements a) to e) are fulfilled:
a) At least one R¹, R², R³, R⁴ and R⁵ is independently selected from CN, partially flurorinated or perfluorinated C₁ to C₈ alkyl, halogen, Cl, F, and at least one remaining R¹, R², R³, R⁴ and R⁵ is selected D or H;
b) R¹ or R² are selected from CN or partially flurorinated or perfluorinated C₁ to C₈ alkyl and at least one remaining R¹ to R⁵ is independently selected from CN, partially flurorinated or perfluorinated C₁ to C₈ alkyl, halogen, Cl or F;
c) R3 is selected from partially flurorinated or perfluorinated C₁ to C₈ alkyl and at least one of R¹, R², R⁴ and R⁵ is independently selected from CN, partially flurorinated or perfluorinated C₁ to C₈ alkyl, halogen, Cl or F;
d) at least two R¹ to R⁵ are independently selected from CN or partially flurorinated or perfluorinated C₁ to C₈ alkyl; or
e) at least one X¹ to X⁵ is N and at least two X¹ to X⁵ are selected from CR¹ to CR⁵;
A² and A³ are independently selected from formula (III) 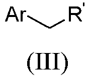
wherein Ar is independently selected from substituted or unsubstituted C₆ to C₁₈ aryl and substituted or unsubstituted C₂ to C₁₈ heteroaryl, wherein the substituents on Ar are independently selected from CN, partially or perfluorinated C₁ to C₆ alkyl, halogen, Cl, F, D; and
R' is selected from Ar, substituted or unsubstituted C₆ to C₁₈ aryl or C₃ to C₁₈ heteroaryl, partially flurorinated or perfluorinated C₁ to C₈ alkyl, halogen, F or CN;
and **characterized by** that the anode layer comprises a first anode sub-layer and a second anode sub-layer, wherein
- the first anode sub-layer comprises a first metal having a work function in the range of ≥ 4 and ≤ 6 eV, whereby the wherein the first metal of the first anode sub-layer is selected from the group comprising Ag, and
- the second anode sub-layer comprises a transparent conductive oxide; and
- the second anode sub-layer is arranged closer to the hole injection layer;
- wherein the anode layer of the organic electronic device comprises in addition a third anode sub-layer comprising a transparent conductive oxide, wherein the third anode sub-layer is arranged between the substrate and the first anode sub-layer;
- whereby the transparent conductive oxide is selected from the group comprising indium tin oxide.

2. The device of claim 1 whereby the hole injection layer comprises a compound of formula (IV) 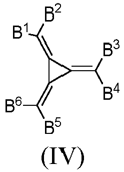
whereby B¹ is selected from formula (V) 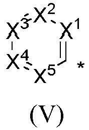
B³ and B⁵ are Ar and B², B⁴ and B⁶ are R'.

3. The device of claim 1 or 2, whereby A² and A³ are identical.

4. The device of any of the claims 1 to 3, whereby A¹ differs from A² and A³.

5. The organic electronic device of any of the claims 1 to 4, whereby the hole injection layer comprises a composition comprising a compound of formula (IV) defined in claim 2 and at least one compound of formula (IVa) to (IVd) 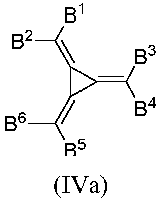 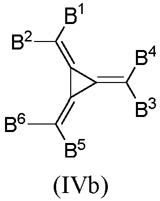 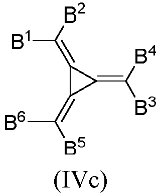 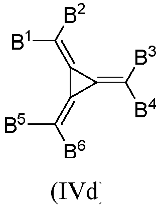 wherein B¹ to B⁶ are defined in claim 2

6. The organic electronic device of any of the claims 1 to 5, wherein LUMO level of compound of formula (I) is selected in the range of ≤ -4.3 eV and ≥ -5.6 eV; wherein the LUMO is calculated with the program package TURBOMOLE V6.5 (TURBOMOLE GmbH, Litzenhardtstrasse 19, 76135 Karlsruhe, Germany), and the optimized geometries and the LUMO energy levels of the molecular structures are determined by applying the hybrid functional B3LYP with a 6-31G* basis set in the gas phase; and further if more than one conformation is viable, the conformation with the lowest total energy is selected.

7. The organic electronic device of any of the claims 1 to 6, wherein the hole injection layer comprises a substantially covalent matrix compound with a molecular weight Mw of ≥ 400 and ≤ 2000 g/mol.

8. The organic electronic device of any of the claims 1 to 7, whereby the substantially covalent matrix compound comprises at least one arylamine compound, diarylamine compound, triarylamine compound, a compound of formula (II) or a compound of formula (III) : 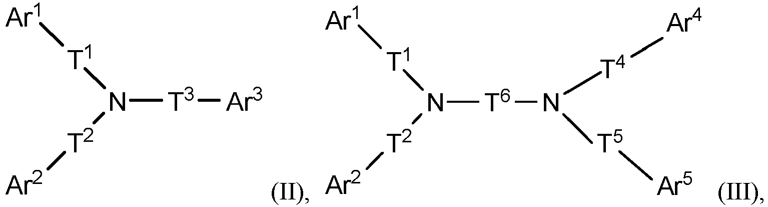 wherein:
T¹, T², T³, T⁴ and T⁵ are independently selected from a single bond, phenylene, biphenylene, terphenylene or naphthenylene, preferably a single bond or phenylene; T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising **H, D,** F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² is selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

9. The organic electronic device of any of the claims 1 to 8, whereby the organic electronic device comprises a hole transport layer, wherein the hole transport layer is arranged between the hole injection layer and the at least one first emission layer and the hole transport layer comprises a substantially covalent matrix compound.

10. A display device comprising an organic electronic device according to any of the claims 1 to 9.

## Patentansprüche

1. Organische elektronische Vorrichtung, umfassend ein Substrat, eine Anodenschicht, eine Kathodenschicht, mindestens eine erste Emissionsschicht und eine Lochinjektionsschicht, wobei die Lochinjektionsschicht zwischen der ersten Emissionsschicht und der Anodenschicht angeordnet ist
und
wobei die Lochinjektionsschicht eine Verbindung der Formel (I) umfasst: 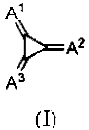
wobei A¹ ausgewählt ist aus Formel (II) 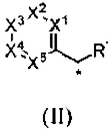
X¹ aus CR¹ oder N ausgewählt ist;
X² aus CR² oder N ausgewählt ist;
X³ aus CR³ oder N ausgewählt ist;
X⁴ aus CR⁴ oder N ausgewählt ist;
X⁵ aus CR⁵ oder N ausgewählt ist;
R¹, R², R³, R⁴ und R⁵, falls vorhanden, unabhängig aus CN, teilweise fluoriertem oder perfluoriertem C₁- bis C₈-Alkyl, Halogen, Cl, F, D oder H ausgewählt sind, wobei dann, wenn eines von R¹, R², R³, R⁴ und R⁵ vorliegt, das entsprechende X¹, X², X³, X⁴ und X⁵ nicht für N steht;
mit der Maßgabe, dass mindestens zwei der folgenden Anforderungen a) bis e) erfüllt sind:
a) mindestens ein R¹, R², R³, R⁴ und R⁵ ist unabhängig aus CN, teilweise fluoriertem oder perfluoriertem C₁- bis C₈-Alkyl, Halogen, Cl, F ausgewählt und mindestens ein verbleibendes R¹, R², R³, R⁴ und R⁵ ist aus D oder H ausgewählt;
b) R¹ oder R² ist aus CN oder teilweise fluoriertem oder perfluoriertem C₁- bis C₈-Alkyl ausgewählt und mindestens ein verbleibendes R¹ bis R⁵ ist unabhängig aus CN, teilweise fluoriertem oder perfluoriertem C₁- bis C₈-Alkyl, Halogen, Cl oder F ausgewählt;
c) R3 ist aus teilweise fluoriertem oder perfluoriertem C₁- bis C₈-Alkyl ausgewählt und mindestens eines von R¹, R², R⁴ und R⁵ ist unabhängig aus CN, teilweise fluoriertem oder perfluoriertem C₁- bis C₈-Alkyl, Halogen, Cl oder F ausgewählt;
d) mindestens zwei R¹ bis R⁵ sind unabhängig aus CN oder teilweise fluoriertem oder perfluoriertem C₁- bis C₈-Alkyl ausgewählt; oder
e) mindestens ein X¹ bis X⁵ steht für N und mindestens zwei X¹ bis X⁵ sind aus CR¹ bis CR⁵ ausgewählt;
A² und A³ unabhängig aus Formel (III) 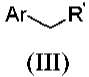
ausgewählt sind, wobei Ar unabhängig aus substituiertem oder unsubstituiertem C₆- bis C₁₈-Aryl und substituiertem oder unsubstituiertem C₂- bis C₁₈-Heteroaryl ausgewählt ist, wobei die Substituenten an Ar unabhängig aus CN, teilweise oder perfluoriertem C₁ bis C₆-Alkyl, Halogen, Cl, F, D ausgewählt sind; und
R' aus Ar, substituiertem oder unsubstituiertem C₆- bis C₁₈-Aryl oder C₃- bis C₁₈-Heteroaryl, teilweise fluroriertem oder perfluoriertem C₁- bis C₈-Alkyl, Halogen, F oder CN ausgewählt ist;
und **dadurch gekennzeichnet, dass** die Anodenschicht eine erste Anodenunterschicht und eine zweite Anodenunterschicht umfasst, wobei
- die erste Anodenunterschicht ein erstes Metall mit einer Austrittsarbeit im Bereich von ≥ 4 und ≤ 6 eV umfasst, wobei das erste Metall der ersten Anodenunterschicht aus der Gruppe umfassend Ag ausgewählt ist, und
- die zweite Anodenunterschicht ein transparentes leitfähiges Oxid umfasst; und
- die zweite Anodenunterschicht näher an der Lochinjektionsschicht angeordnet ist;
- wobei die Anodenschicht der organischen elektronischen Vorrichtung zusätzlich eine dritte Anodenunterschicht umfasst, die ein transparentes leitfähiges Oxid umfasst, wobei die dritte Anodenunterschicht zwischen dem Substrat und der ersten Anodenunterschicht angeordnet ist;
- wobei das transparente leitfähige Oxid aus der Gruppe umfassend Indiumzinnoxid ausgewählt ist.

2. Vorrichtung nach Anspruch 1, wobei die Lochinjektionsschicht eine Verbindung der Formel (I) umfasst: 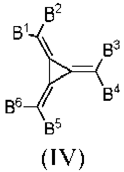
wobei B¹ aus Formel (V) 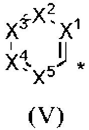
ausgewählt ist, B³ und B⁵ für Ar stehen und B², B⁴ und B⁶ für R' stehen.

3. Vorrichtung nach Anspruch 1 oder 2, wobei A² und A³ gleich sind.

4. Vorrichtung nach einem der Ansprüche 1 bis 3, wobei A¹ von A² und A³ verschieden ist.

5. Organische elektronische Vorrichtung nach einem der Ansprüche 1 bis 4, wobei die Lochinjektionsschicht eine Zusammensetzung umfasst, die eine Verbindung der Formel (IV) gemäß Anspruch 2 und mindestens eine Verbindung der Formel (IVa) bis (IVd) umfasst, 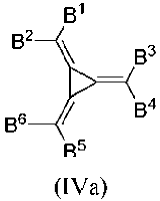 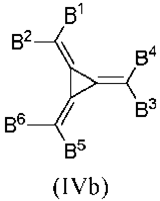 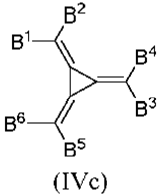 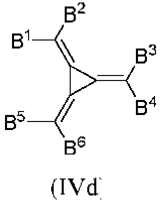 wobei B¹ bis B⁶ in Anspruch 2 definiert sind.

6. Organische elektronische Vorrichtung nach einem der Ansprüche 1 bis 5, wobei das LUMO-Niveau der Verbindung der Formel (I) im Bereich von ≤ -4,3 eV und ≥ -5,6 eV ausgewählt ist;
wobei das LUMO mit dem Programmpaket TURBOMOLE V6.5 (TURBOMOLE GmbH, Litzenhardtstrasse 19, 76135 Karlsruhe, Deutschland) berechnet wird und die optimierten Geometrien und die LUMO-Energieniveaus der Molekülstrukturen durch Anwenden des Hybridfunktionals B3LYP mit einem 6-31G*-Basissatz in der Gasphase berechnet wird und ferner dann, wenn mehr als eine Konformation existenzfähig ist, die Konformation mit der niedrigsten Gesamtenergie ausgewählt wird.

7. Organische elektronische Vorrichtung nach einem der Ansprüche 1 bis 6, wobei die Lochinjektionsschicht eine weitgehend kovalente Matrixverbindung mit einem Molekulargewicht Mw von ≥ 400 und ≤ 2000 g/mol umfasst.

8. Organische elektronische Vorrichtung nach einem der Ansprüche 1 bis 7, wobei die weitgehend kovalente Matrixverbindung mindestens eine Arylaminverbindung, Diarylaminverbindung, Triarylaminverbindung, eine Verbindung der Formel (II) oder eine Verbindung der Formel (III) umfasst: 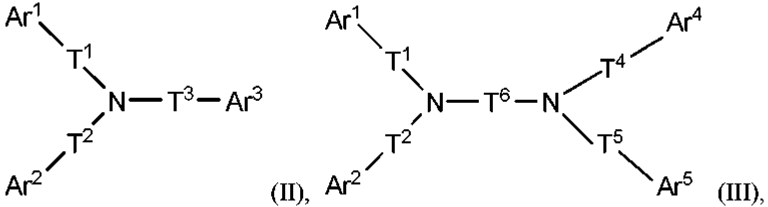 wobei:
T¹, T², T³, T⁴ und T⁵ unabhängig aus einer Einfachbindung, Phenylen, Biphenylen, Terphenylen oder Naphthenylen, vorzugsweise einer Einfachbindung oder Phenylen, ausgewählt sind; T⁶ für Phenylen, Biphenylen, Terphenylen oder Naphthenylen steht;
Ar¹, Ar², Ar³, Ar⁴ und Ar⁵ unabhängig aus substituiertem oder unsubstituiertem C₆- bis C₂₀-Aryl oder substituiertem oder unsubstituiertem C₃- bis C₂₀-Heteroarylen, substituiertem oder unsubstituiertem Biphenylen, substituiertem oder unsubstituiertem Fluoren, substituiertem 9-Fluoren, substituiertem 9,9-Fluoren, substituiertem oder unsubstituiertem Naphthalin, substituiertem oder unsubstituiertem Anthracen, substituiertem oder unsubstituiertem Phenanthren, substituiertem oder unsubstituiertem Pyren, substituiertem oder unsubstituiertem Perylen, substituiertem oder unsubstituiertem Triphenylen, substituiertem oder unsubstituiertem Tetracen, substituiertem oder unsubstituiertem Tetraphen, substituiertem oder unsubstituiertem Dibenzofuran, substituiertem oder unsubstituiertem Dibenzothiophen, substituiertem oder unsubstituiertem Xanthen, substituiertem oder unsubstituiertem Carbazol, substituiertem 9-Phenylcarbazol, substituiertem oder unsubstituiertem Azepin, substituiertem oder unsubstituiertem Dibenzo[b,f]azepin, substituiertem oder unsubstituiertem 9,9'-Spirobi[fluoren], substituiertem oder unsubstituiertem Spiro[fluoren-9,9'-xanthen] oder einem substituierten oder unsubstituierten aromatischen anellierten Ringsystem mit mindestens drei substituierten oder unsubstituierten aromatischen Ringen, die aus der Gruppe umfassend 5-gliedrige substituierte oder unsubstituierte Nichthetero- oder substituierte oder unsubstituierte Heteroringe, substituierte oder unsubstituierte 6-gliedrige Ringe und/oder substituierte oder unsubstituierte 7-gliedrige Ringe ausgewählt sind, substituiertem oder unsubstituiertem Fluoren oder einem anellierten Ringsystem mit 2 bis 6 substituierten oder unsubstituierten 5- bis 7-gliedrigen Ringen ausgewählt sind und die Ringe aus der Gruppe umfassend (i) einen ungesättigten 5- bis 7-gliedrigen Ring eines Heterocyclus, (ii) einen 5- bis 6-gliedrigen Ring eines aromatischen Heterocyclus, (iii) einen ungesättigten 5-bis 7-gliedrigen Ring eines Nichtheterocyclus, (iv) einen 6-gliedrigen Ring eines aromatischen Nichtheterocyclus ausgewählt sind;
wobei
die Substituenten von Ar¹, Ar², Ar³, Ar⁴ und Ar⁵ gleich oder verschieden aus der Gruppe umfassend H, D, F, C(-O)R², CN, Si(R²)₃, P(-O)(R²)₂, OR², S(-O)R², S(-O)₂R², substituiertes oder unsubstituiertes geradkettiges Alkyl mit 1 bis 20 Kohlenstoffatomen, substituiertes oder unsubstituiertes verzweigtes Alkyl mit 1 bis 20 Kohlenstoffatomen, substituiertes oder unsubstituiertes cyclisches Alkyl mit 3 bis 20 Kohlenstoffatomen, substituierte oder unsubstituierte Alkenyl- oder Alkinylgruppen mit 2 bis 20 Kohlenstoffatomen, substituierte oder unsubstituierte Alkoxygruppen mit 1 bis 20 Kohlenstoffatomen, substituierte oder unsubstituierte aromatische Ringsysteme mit 6 bis 40 aromatischen Ringatomen und substituierte oder unsubstituierte heteroaromatische Ringsysteme mit 5 bis 40 aromatischen Ringatomen, unsubstituiertes C₆- bis C₁₈-Aryl, unsubstituiertes C₃- bis C₁₈-Heteroaryl, ein anelliertes Ringsystem mit 2 bis 6 unsubstituierten 5-bis 7-gliedrigen Ringen, wobei die Ringe aus der Gruppe umfassend einen ungesättigten 5- bis 7-gliedrigen Ring eines Heterocyclus, einen 5- bis 6-gliedrigen Ring eines aromatischen Heterocyclus, einen ungesättigten 5- bis 7-gliedrigen Ring eines Nichtheterocyclus und einen 6-gliedrigen Ring eines aromatischen Nichtheterocyclus ausgewählt sind, ausgewählt sind,
wobei R² aus H, D, geradkettigem Alkyl mit 1 bis 6 Kohlenstoffatomen, verzweigtem Alkyl mit 1 bis 6 Kohlenstoffatomen, cyclischem Alkyl mit 3 bis 6 Kohlenstoffatomen, Alkenyl- oder Alkinylgruppen mit 2 bis 6 Kohlenstoffatomen, C₆- bis C₁₈-Aryl oder C₃- bis C₁₈-Heteroaryl ausgewählt ist.

9. Organische elektronische Vorrichtung nach einem der Ansprüche 1 bis 8, wobei die organische elektronische Vorrichtung eine Lochtransportschicht umfasst, wobei die Lochtransportschicht zwischen der Lochinjektionsschicht und der mindestens einen ersten Emissionsschicht angeordnet ist und die Lochtransportschicht eine weitgehend kovalente Matrixverbindung umfasst.

10. Anzeigevorrichtung, umfassend eine organische elektronische Vorrichtung nach einem der Ansprüche 1 bis 9.

## Revendications

1. Dispositif électronique organique comprenant un substrat, une couche d'anode, une couche de cathode, au moins une première couche d'émission, et une couche d'injection de trous, dans lequel la couche d'injection de trous est disposée entre la première couche d'émission et la couche d'anode,
et
moyennant quoi la couche d'injection de trous comprend un composé de formule (I) 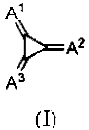
moyennant quoi A¹ est choisi parmi la formule (II) 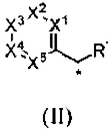
X¹ est choisi parmi CR¹ et N ;
X² est choisi parmi CR² et N ;
X³ est choisi parmi CR³ et N ;
X⁴ est choisi parmi CR⁴ et N ;
X⁵ est choisi parmi CR⁵ et N ;
R¹, R², R³, R⁴ et R⁵, s'ils sont présents sont choisis indépendamment parmi CN, alkyle en C₁ à C₈ partiellement fluoré ou perfluoré, halogène, Cl, F, D ou H, moyennant quoi lorsque l'un quelconque des R¹, R², R³, R⁴ et R⁵ est présent, alors les X¹, X², X³, X⁴ et X⁵ correspondants ne sont pas N ;
à condition qu'au moins deux des conditions suivantes a) à e) soient remplies :
a) au moins un parmi R¹, R², R³, R⁴ et R⁵ est indépendamment choisi parmi CN, alkyle en C₁ à C₈ partiellement fluoré ou perfluoré, halogène, Cl, F, et au moins un R¹, R², R³, R⁴ et R⁵ restant est choisi parmi D ou H ;
b) R¹ ou R² sont choisis parmi CN ou alkyle en C₁ à C₈ partiellement fluoré ou perfluoré et au moins un R¹ à R⁵ restant est choisi indépendamment parmi CN, alkyle en C₁ à C₈ partiellement fluoré ou perfluoré, halogène, Cl ou F ;
c) R3 est choisi parmi alkyle en C₁ à C₈ partiellement fluoré ou perfluoré et au moins l'un parmi R¹, R², R⁴ et R⁵ est choisi indépendamment parmi CN, alkyle en C₁ à C₈ partiellement fluoré ou perfluoré, halogène, Cl ou F ;
d) au moins deux R¹ à R⁵ sont choisis indépendamment parmi CN ou alkyle en C₁ à C₈ partiellement fluoré ou perfluoré ; ou
e) au moins un X¹ à X⁵ est N et au moins deux X¹ à X⁵ sont choisis parmi CR¹ à CR⁵ ;
A² et A³ sont indépendamment choisis parmi la formule (III) 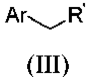
dans laquelle Ar est choisi indépendamment parmi aryle en C₆ à C₁₈ substitué ou non substitué et hétéroaryle en C₂ à C₁₈, substitué ou non substitué, dans laquelle les substituants sur Ar sont choisis indépendamment parmi CN, alkyle en C₁ à C₆ partiellement ou perfluoré, halogène, Cl, F, D ; et
R' est choisi parmi Ar, aryle en C₆ à C₁₈ substitué ou non substitué ou hétéroaryle en C₃ à C₁₈, alkyle en C₁ à C₈ partiellement fluoré ou perfluoré, halogène, F ou CN ;
et **caractérisé en ce que** la couche d'anode comprend une première sous-couche d'anode et une seconde sous-couche d'anode, dans lequel
- la première sous-couche d'anode comprend un premier métal ayant un travail d'extraction dans la plage de ≥ 4 et ≤ 6 eV, moyennant quoi le premier métal de la première sous-couche d'anode est choisi dans le groupe comprenant Ag, et
- la deuxième sous-couche d'anode comprend un oxyde conducteur transparent ; et
- la deuxième sous-couche d'anode est agencée plus proche de la couche d'injection de trous ;
- dans lequel la couche d'anode du dispositif électronique organique comprend en outre une troisième sous-couche d'anode comprenant un oxyde conducteur transparent, dans lequel la troisième sous-couche d'anode est disposée entre le substrat et la première sous-couche d'anode ;
- moyennant quoi l'oxyde conducteur transparent est sélectionné dans le groupe comprenant l'oxyde d'indium-étain.

2. Dispositif selon la revendication 1, moyennant quoi la couche d'injection de trous comprend un composé de formule (IV) 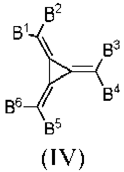
moyennant quoi B¹ est choisi parmi la formule (V) 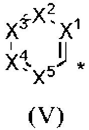
B³ et B⁵ sont Ar et B², B⁴ et B⁶ sont R'.

3. Dispositif selon la revendication 1 ou 2, moyennant quoi A² et A³ sont identiques.

4. Dispositif selon l'une quelconque des revendications 1 à 3, moyennant quoi A¹ diffère de A² et A³.

5. Dispositif électronique organique selon l'une quelconque des revendications 1 à 4, moyennant quoi la couche d'injection de trous comprend une composition comprenant un composé de formule (IV) défini dans la revendication 2 et au moins un composé de formule (IVa) à (IVd) 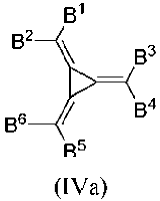 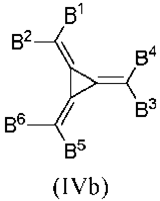 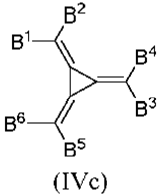 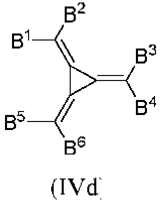 dans laquelle B¹ à B⁶ sont définis selon la revendication 2.

6. Dispositif électronique organique selon l'une quelconque des revendications 1 à 5, dans lequel le niveau de LUMO du composé de formule (I) est choisi dans la plage de ≤ -4,3 eV et ≥ -5,6 eV ;
dans laquelle la LUMO est calculée avec le progiciel TURBOMOLE V6.5 (TURBOMOLE GmbH, Litzenhardtstrasse 19, 76135 Karlsruhe, Allemagne), et les géométries optimisées et les niveaux d'énergie de LUMO des structures moléculaires sont déterminés en appliquant le B3LYP fonctionnel hybride avec un ensemble de base 6-31G* défini dans la phase gazeuse ; et en outre, si plus d'une conformation est viable, la conformation ayant l'énergie totale la plus faible est sélectionnée.

7. Dispositif électronique organique selon l'une quelconque des revendications 1 à 6, dans lequel la couche d'injection de trous comprend un composé matriciel sensiblement covalent ayant un poids moléculaire Mw de ≥ 400 et ≤ 2 000 g/mol.

8. Dispositif électronique organique selon l'une quelconque des revendications 1 à 7, moyennant quoi le composé matriciel sensiblement covalent comprend au moins un composé arylamine, composé diarylamine, composé triarylamine, un composé de formule (II) ou un composé de formule (III) : 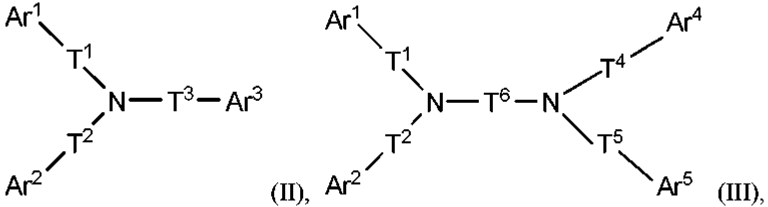 dans lesquelles :
T¹, T², T³, T⁴ et T⁵ sont choisis indépendamment parmi une simple liaison, phénylène, biphénylène, terphénylène ou naphténylène, préférablement une simple liaison ou phénylène ; T⁶ est phénylène, biphénylène, terphénylène ou naphténylène ;
Ar¹, Ar², Ar³, Ar⁴ et Ar⁵ sont choisis indépendamment parmi aryle en C₆ à C₂₀ substitué ou non substitué, ou hétéroarylène en C₃ à C₂₀ substitué ou non substitué, biphénylène substitué ou non substitué, fluorène substitué ou non substitué, 9-fluorène substitué, 9,9-fluorène substitué, naphtalène substitué ou non substitué, anthracène substitué ou non substitué, phénanthrène substitué ou non substitué, pyrène substitué ou non substitué, pérylène substitué ou non substitué, triphénylène substitué ou non substitué, tétracène substitué ou non substitué, tétraphène substitué ou non substitué, dibenzofurane substitué ou non substitué, dibenzothiophène substitué ou non substitué, xanthène substitué ou non substitué, carbazole substitué ou non substitué, 9-phénylcarbazole substitué, azépine substituée ou non substituée, dibenzo[b,f]azépine substituée ou non substituée, 9,9'-spirobi[fluorène] substitué ou non substitué, spiro[fluorène-9,9'-xanthène] substitué ou non substitué, ou un système à anneaux fusionnés aromatiques substitués ou non substitués comprenant au moins trois anneaux aromatiques substitués ou non substitués choisis dans le groupe comprenant des anneaux à 5 chaînons non hétéro substitués ou non substitués, hétéro substitués ou non substitués, des anneaux à 6 chaînons substitués ou non substitués et/ou des anneaux à 7 chaînons substitués ou non substitués, fluorène substitué ou non substitué, ou un système à anneaux fusionnés comprenant 2 à 6 anneaux à 5 à 7 chaînons substitués ou non substitués et les anneaux sont choisis dans le groupe comprenant (i) un anneau à 5 à 7 chaînons insaturé d'un hétérocycle, (ii) 5 à 6 chaînons d'un hétérocycle aromatique, (iii) un anneau à 5 à 7 chaînons insaturé d'un non hétérocycle, (iv) un anneau à 6 chaînons d'un non hétérocycle aromatique ;
dans laquelle
les substituants de Ar¹, Ar², Ar³, Ar⁴ et Ar⁵ sont choisis identiques ou différents dans le groupe comprenant H, D, F, C(-O)R², CN, Si(R²)₃, P(-O)(R²)₂, OR², S(-O)R², S(-O)₂R², alkyle à chaîne linéaire substitué ou non substitué ayant 1 à 20 atomes de carbone, alkyle ramifié substitué ou non substitué ayant 1 à 20 atomes de carbone, alkyle cyclique substitué ou non substitué ayant 3 à 20 atomes de carbone, groupes alcényle ou alcynyle substitués ou non substitués ayant 2 à 20 atomes de carbone, groupes alcoxy substitués ou non substitués ayant 1 à 20 atomes de carbone, systèmes à anneaux aromatiques substitués ou non substitués ayant 6 à 40 atomes d'anneaux aromatiques, et systèmes à anneaux hétéroaromatiques substitués ou non substitués ayant 5 à 40 atomes d'anneaux aromatiques, aryle en C₆ à C₁₈ non substitué, hétéroaryle en C₃ à C₁₈ non substitué, un système à anneaux fusionnés comprenant 2 à 6 anneaux à 5 à 7 chaînons non substitués et les anneaux sont choisis dans le groupe comprenant un anneau à 5 à 7 chaînons insaturé d'un hétérocycle, 5 à 6 chaînons d'un hétérocycle aromatique, un anneau à 5 à 7 chaînons insaturé d'un non hétérocycle, et un anneau à 6 chaînons d'un non hétérocycle aromatique,
dans lequel R² est choisi parmi H, D, alkyle à chaîne linéaire ayant 1 à 6 atomes de carbone, alkyle ramifié ayant 1 à 6 atomes de carbone, alkyle cyclique ayant 3 à 6 atomes de carbone, groupes alcényle ou alcynyle ayant 2 à 6 atomes de carbone, aryle en C₆ à C₁₈ ou hétéroaryle en C₃ à C₁₈.

9. Dispositif électronique organique selon l'une quelconque des revendications 1 à 8, moyennant quoi le dispositif électronique organique comprend une couche de transport de trous, dans lequel la couche de transport de trous est disposée entre la couche d'injection de trous et l'au moins une première couche d'émission et la couche de transport de trous comprend un composé matriciel sensiblement covalent.

10. Dispositif d'affichage comprenant un dispositif électronique organique selon l'une quelconque des revendications 1 à 9.
